(19) Europäisches Patentamt
European Patent Office
Office européen des brevets

(11) Veröffentlichungsnummer: **0 061 661**
**A1**

# (12) EUROPÄISCHE PATENTANMELDUNG

(21) Anmeldenummer: 82102200.1

(22) Anmeldetag: 18.03.82

(51) Int. Cl.³: C 07 D 251/16
C 07 D 251/22, C 07 D 251/46
C 07 D 239/42, C 07 D 239/46
C 07 D 239/52, C 07 D 253/06
A 01 N 47/36

(30) Priorität: 24.03.81 DE 3111451

(43) Veröffentlichungstag der Anmeldung: 06.10.82 Patentblatt 82/40

(84) Benannte Vertragsstaaten: AT BE CH DE FR GB IT LI LU NL SE

(71) Anmelder: HOECHST AKTIENGESELLSCHAFT
Postfach 80 03 20
D-6230 Frankfurt/Main 80(DE)

(72) Erfinder: Willms, Lothar, Dr.
Grüner Weg 4
D-5463 Unkel(DE)

(72) Erfinder: Mildenberger, Hilmar, Dr.
Fasanenstrasse 24
D-6233 Kelkheim (Taunus)(DE)

(72) Erfinder: Günther, Dieter, Dr.
Nachtigallenweg 1A
D-6233 Kelkheim(Taunus)(DE)

(72) Erfinder: Bauer, Klaus, Dr.
Kolpingstrasse 7
D-6054 Rodgau(DE)

(72) Erfinder: Bürstell, Helmut, Dr.
Am Hohlacker 65
D-6000 Frankfurt am Main 50(DE)

(72) Erfinder: Bieringer, Hermann, Dr.
Eichenweg 26
D-6239 Eppstein/Taunus(DE)

## (54) Heterocyclisch substituierte (Halogen)Alkyl- und -Alkoxysulfonylharnstoffe, Verfahren zu ihrer Herstellung und ihre Verwendung in der Landwirtschaft.

(57) Verbindungen der Formel

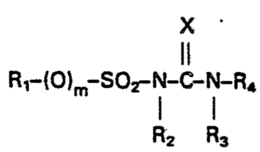

worin $R_1$ einen gegebenenfalls mit Halogen substituierten und/oder durch Sauerstoff unterbrochenen aliphatischen Rest, $R_2$ und $R_3$ H oder Alkyl, X Sauerstoff oder Schwefel, in 0 oder 1 und $R_4$ einen gegebenenfalls substituierten heterocyclischen Sechsring mit 2-3 N-Atomen bedeuten, sind wirksame Herbizide und Wachstumsregulatoren.

EP 0 061 661 A1

Heterocyclisch substituierte (Halogen)Alkyl- und -Alkoxysulfonylharnstoffe, Verfahren zu ihrer Herstellung und ihre Verwendung in der Landwirtschaft

Es ist bereits bekannt, daß heterocyclisch substituierte Phenylsulfonylharnstoffe, wie z.B. N-(4-Chlor-6-i-propylamino-1,3,5-triazin-2-yl)-N-i-propyl-N'-(4-chlorphenylsulfonyl)-harnstoff, herbizide oder pflanzenwuchsregulierende Eigenschaften aufweisen (vgl. NL-PS 121 788, DE-OS 27 15 786, EP 1 485, EP 1 514, EP 1 515, EP 4 163, EP 7 687, EP 9 419, EP 10 560).

Es wurde nun gefunden, daß auch heterocyclisch substituierte (Halogen)Alkyl- bzw. Alkoxysulfonylharnstoffe als Herbizide und Pflanzenwachstumsregulatoren geeignet sind.

Gegenstand der vorliegenden Erfindung sind demnach Verbindungen der Formel I

$$R_1-(O)_m-SO_2-\underset{R_2}{N}-\overset{X}{\overset{\|}{C}}-\underset{R_3}{N}-R_4 \qquad (I)$$

worin

$R_1$ einen gegebenenfalls durch bis zu 6 Halogenatome substituierten gesättigten oder ungesättigten, verzweigten oder unverzweigten aliphatischen Rest mit bis zu 10 C-Atomen, der ggf. durch Sauerstoff unterbrochen sein kann,

$R_2$, $R_3$ H oder $(C_1-C_4)$-Alkyl

X O oder S;

m 0 oder 1;

$R_4$ einen 2-3 Stickstoffatome enthaltenden heterocyclischen Sechsring, der ggf. 1-3 fach durch Halogen, $NO_2$, CN, CHO, $(C_1-C_4)$-Alkylamino, $(C_1-C_4)$Dialkylamino, einen (gegebenenfalls durch Halogen, $(C_1-C_3)$-Alkoxy,

($C_1$-$C_3$)-Alkylthio, ($C_1$-$C_3$)-Alkylamino, ($C_1$-$C_3$)-Dialkylamino oder ($C_1$-$C_4$)Alkoxycarbonyl substituierten) ($C_1$-$C_4$)-Alkylrest, einen (gegebenenfalls durch Halogen oder ($C_1$-$C_4$)Alkoxycarbonyl substituierten) ($C_1$-$C_4$)-Alkoxy- oder ($C_1$-$C_4$)-Alkylthiorest oder ($C_1$-$C_4$)-Alkoxycarbonyl substituiert ist,

bedeuten, sowie, falls $R_2$ Wasserstoff bedeutet, deren physiologisch verträgliche Salze mit Basen.

Unter "aliphatischen Resten" in $R_1$-Stellung sind Alkylreste, Alkenylreste mit einfacher oder konjugierter Doppelbindung oder Alkinylreste zu verstehen. "Halogen" bedeutet bevorzugt Fluor, Chlor oder Brom. Reste mit mindestens 3 C-Atomen zeichnen sich durch besonders starke herbizide Wirkung aus.

Als Beispiele für erfindungsgemäße heterocyclisch substituierte Sulfonylharnstoffe der Formel I seien die folgenden genannt:

N-(4-Methoxy-6-methyl-pyrimidin-2-yl)-N'-(2,2,2-trichloreth-1-oxysulfonyl)-harnstoff;
N-(4-Methoxy-6-methyl-1,3,5-triazin-2-yl)-N'-(2,2,2-trifluoreth-1-oxysulfonyl)-harnstoff;
N-(2,6-Dimethyl-5-chlor-pyrimidin-4-yl)-N'-(methoxysulfonyl)-harnstoff;
N-(4-Methyl-5-ethoxycarbonyl-pyrimidin-2-yl)-N'-(isobutyloxysulfonyl)-harnstoff;
N-(4-Methoxy-6-methylthio-pyrimidin-2-yl)-N'-(2-chloreth-1-yl-sulfonyl)-harnstoff;
N-(4,6-Dichlor-pyrimidin-2-yl)-N'-(2-chloreth-1-yl-sulfonyl)-harnstoff;
N-(4-Methyl-6-methylthio-1,3,5-triazin-2-yl)-N'-(1,2-dichlor-n-prop-1-yl-sulfonyl)-harnstoff;
N-(4-Methyl-6-dimethylamino-1,3,5-triazin-2-yl)-N'-(1,2-dichlor-n-prop-1-yl-sulfonyl)-harnstoff;

N-(4-Methoxy-6-methyl-1,3,5-triazin-2-yl)-N'-(1-methyl-2-chlor-n-prop-1-yl-sulfonyl)-harnstoff;

N-(4-Methoxy-6-methyl-pyrimidin-2-yl)-N'-(2-chlor-3-methoxy-n-prop-1-yl-sulfonyl)-harnstoff;

N-(5,6-Dimethyl-1,2,4-triazin-3-yl)-N'-(1,2,2-trichlor-eth-1-yl-sulfonyl)-harnstoff;

N-(4,6-Dimethoxy-5-chlor-pyrimidin-2-yl)-N'-(2-chlor-n-but-1-yl-sulfonyl)-harnstoff;

N-(4,6-Dimethyl-5-nitro-pyrimidin-2-yl)-N'-(2,4,6,6-tetrachlor-n-hex-1-yl-sulfonyl)-harnstoff;

N-(4,5-Dimethyl-6-methoxy-pyrimidin-2-yl)-N'-(2-chlor-n-hex-1-yl-sulfonyl)-harnstoff;

N-(4-Methyl-6-diethylamino-1,3,5-triazin-2-yl)-N'-(vinylsulfonyl)-harnstoff;

N-(4,6-Dimethyl-5-brom-pyrimidin-2-yl)-N'-(prop-1-en-1-yl-sulfonyl)-harnstoff;

N-(4-Methyl-5-nitro-6-chlor-pyrimidin-2-yl)-N'-(3-chlor-prop-1-en-1-yl-sulfonyl)-harnstoff;

N-(4-Methyl-6-ethoxymethyl-pyrimidin-2-yl)-N'-(4,4-dichlor-but-1-en-1-yl-sulfonyl)-harnstoff;

N-(4,6-Dimethoxy-1,3,5-triazin-2-yl)-N-methyl-N'-(2-chlor-vinylsulfonyl)-harnstoff;

N-(4,6-Diethyl-pyrimidin-2-yl)-N'-(prop-1-en-1-yl-sulfonyl)-harnstoff, -Natriumsalz;

N-(Pyrimidin-2-yl)-N'-(prop-1-en-1-yl-sulfonyl)-thioharnstoff;

N-(4-Chlor-6-isopropylamino-1,3,5-triazin-2-yl)-N-isopropyl-N'-(prop-1-en-1-yl-sulfonyl)-harnstoff;

N-(5,6-Dimethoxy-1,2,4-triazin-3-yl)-N'-(n-prop-1-oxysulfonyl)-harnstoff;

N-(4,6-Dimethoxy-5-fluor-pyrimidin-2-yl)-N'-(n-heptyl-1-oxysulfonyl)-harnstoff;

N-(4,6-Dimethyl-5-jod-pyrimidin-2-yl)-N'-(2-methoxy-ethoxysulfonyl)-harnstoff;

N-(4,6-Dichlor-5-(2-chlorethyl)-pyrimidin-2-yl)-N'-(1,3-dichlor-isopropyl-2-oxysulfonyl)-harnstoff;

N-(4-Methoxy-5-nitro-6-methyl-pyrimidin-2-yl)-N'-(1,1,1,3,3,3-hexachlor-isopropyl-2-oxysulfonyl)-harnstoff;
N-(4-Trifluormethyl-6-methyl-pyrimidin-2-yl)-N'-(2,2-dichlor-ethyl-sulfonyl)-harnstoff;
N-(4,5-Dimethyl-6-methoxyethoxy-pyrimidin-2-yl)-N'-(1,2-dichlor-ethyl-sulfonyl)-harnstoff;
N-(4-Methoxymethyl-5-methyl-6-ethoxy-pyrimidin-2-yl)-N'-(2,3-dichlor-n-prop-1-yl-sulfonyl)-harnstoff;
N-(4-(1-Ethoxycarbonyl-ethoxy)-6-methyl-pyrimidin-2-yl)-N'-(1,2-dibrom-n-prop-1-yl-sulfonyl)-harnstoff;
N-(4-(Methoxycarbonyl-methoxy)-6-methyl-1,3,5-triazin-2-yl)-N'-(2-chlor-n-pent-1-yl-sulfonyl)-harnstoff;
N-(4,6-Diethylmercapto-pyrimidin-2-yl)-N'-(2-chlor-2-trifluormethyl-eth-1-yl-sulfonyl)-harnstoff;
N-(4-Methyl-6-(2-bromethoxy)-pyrimidin-2-yl)-N'-(2,3,4-trichlor-n-but-1-yl-sulfonyl)-harnstoff;
N-(4-Methoxy-6-methyl-1,3,5-triazin-2-yl)-N'-(3-chlor-2-chlormethyl-prop-1-yl-sulfonyl)-harnstoff;
N-(4,6-Dimethylmercapto-1,3,5-triazin-2-yl)-N'-(2-chlor-2-chlormethyl-prop-1-yl-sulfonyl)-harnstoff;
N-(4-Methyl-pyrimidin-2-yl)-N'-(2,3-dichlor-n-but-1-yl-sulfonyl)-harnstoff;
N-(4-Methoxy-5-n-butyl-6-methyl-pyrimidin-2-yl)-N'-(3-chlor-n-but-1-en-1-yl-sulfonyl)-harnstoff;
N-(4,6-Dimethoxy-1,3,5-triazin-2-yl)-N'-(1-methyl-2,3-dichlor-n-prop-1-yl-sulfonyl)-harnstoff;
N-(2,6-Dimethyl-pyrimidin-4-yl)-N'-(2-chlor-3-brom-n-but-1-yl-sulfonyl)-harnstoff;
N-(5,6-Dimethyl-1,2,4-triazin-2-yl)-N'-(1-methyl-n-prop-1-en-1-yl-sulfonyl)-harnstoff;
N-(5-Chlor-pyrimidin-2-yl)-N'-(2-chlor-2,4-dibrom-n-but-1-yl-sulfonyl)-harnstoff.

Die neuen Verbindungen der allgemeinen Formel I lassen sich aus an sich bekannten bzw. nach bekannten Verfahren hergestellten Ausgangsmaterialien synthetisieren. Die Herstellungsverfahren sind dadurch gekennzeichnet, daß man

a) Verbindungen der Formel

$$R_1 - (O)_m - SO_2 - N = C = O \qquad (II),$$

oder

$$R_1 - (O)_m - SO_2 - \underset{\displaystyle R_2}{\underset{|}{N}} - \overset{\displaystyle X}{\overset{\|}{C}} - Cl \qquad (III)$$

mit Verbindungen der Formel

$$\underset{\displaystyle R_3}{\underset{|}{HN}} - R_4 \qquad (IV)$$

oder

b) Verbindungen der Formel

$$R_1 - (O)_m - SO_2 - \underset{\displaystyle R_2}{\underset{|}{NH}} \qquad (V)$$

mit Verbindungen der Formel

$$S = C = N - R_4 \qquad (VI)$$

oder

$$Cl - \overset{\displaystyle X}{\overset{\|}{C}} - \underset{\displaystyle R_3}{\underset{|}{N}} - R_4 \qquad (VII)$$

umsetzt, wobei in Formel VII $R_3$ für $(C_1-C_4)$-Alkyl steht, und gewünschtenfalls die erhaltenen Verbindungen der Formel I durch Abspaltung von Halogenwasserstoff, Anlagerung von Halogen an vorhandene Mehrfachbindungen, Alkylierung in $R_2$-Position oder Salzbildung in andere Verbindungen der Formel I überführt.

Zu a) Die Umsetzung der Verbindungen II bzw. III und IV erfolgt vorzugsweise in inerten aprotischen Lösungsmitteln wie z.B. Acetonitril, Dichlormethan, Toluol, Tetrahydrofuran oder Dioxan bei Temperaturen zwischen 0°C und der Siedetemperatur des Lösungsmitteln. Bei Anwendung von Ausgangsstoffen der Formel III arbeitet man in Gegenwart eines Säureakzeptors wie z.B. Kaliumcarbonat, Pyridin oder Triethylamin.

Zu b) Bei der Umsetzung der Verbindungen V mit VI bzw. VII arbeitet man gleichfalls vorzugsweise in den oben genannten inerten Lösungsmitteln unter Zusatz basischer Verbindungen, wie z.B. Kaliumcarbonat, Pyridin oder Triethylamin bei Temperaturen zwischen 0°C und der Siedetemperatur des Lösungsmittels.

Die nachträgliche Abspaltung von Halogenwasserstoff (HCl, HBr) aus halogenhaltigen Resten $R_1$ erfolgt in bekannter Weise, z.B. mit Alkalialkoholat, alkoholischer Natron- oder Kalilauge, Triethylamin oder anderen säureabspaltenden Mitteln, ggf. in Gegenwart eines weiteren inerten Lösungs- oder Verdünnungsmittels (z.B. Toluol) bei Temperaturen zwischen Raum- und Siedetemperatur.

An vorhandene oder nachträglich gebildete Mehrfachbindungen in $R_1$-Position kann man Halogen ($Cl_2$, $Br_2$) oder Halogenwasserstoff in gleichfalls bekannter Weise anlagern und so ggf. zu neuen Verbindungen der Formel I gelangen. Die Bromierung bzw. Chlorierung wird in inerten organischen Lösungsmitteln wie z.B. Dichlormethan oder Chloroform unter Belichtung - beispielsweise mit ultraviolettem Licht - oder in Gegenwart von radikalisch zerfallenden Verbindungen - z.B. Azodiisobutyronitril - bei Temperaturen zwischen 0°C und der Siedetemperatur des Lösungsmittels durchgeführt. Die Anlagerung von Halogenwasserstoff gelingt in Gegenwart inerter Lösungsmittel (z.B. Toluol) mittels gasförmigem HCl oder HBr bei niedrigen Temperaturen, ggf. in Gegenwart eines Peroxidkatalysators.

Zur nachträglichen Alkylierung in $R_2$-Stellung arbeitet man vorzugsweise in inerten aprotischen Lösungsmitteln wie z.B. Dioxan oder Dimethylformamid unter Zusatz einer anorganischen Base, z.B. Natriumhydrid oder Kaliumcarbonat, bei Temperaturen von 20°C bis zur Siedetemperatur des Lösungsmittels. Als Alkylierungsmittel werden z.B. Dimethylsulfat, Methyljodid oder Ethylbromid eingesetzt.

Verbindungen der Formel I, in denen $R_2$ Wasserstoff bedeutet, können Salze bilden, bei denen H durch ein für die Landwirtschaft geeignetes Kation ersetzt ist. Diese Salze sind im allgemeinen Metall-, Ammonium- oder organische Aminsalze und werden vorzugsweise in inerten Lösungsmitteln wie z.B. Wasser, Methanol oder Aceton bei Temperaturen von 20 - 100°C hergestellt. Geeignete Basen zur Herstellung der erfindungsgemäßen Salze sind z.B. Kaliumcarbonat, Ammoniak oder Ethanolamin.

Die Ausgangsstoffe der Formel IV sind bekannt oder lassen sich nach im Prinzip bekannten Verfahren herstellen, z.B. durch Zyklisierung entsprechender Guanidinderivate mit entsprechend substituierten 1,3-Diketonen (vgl. z.B. "The Chemistry of Heterocyclic compounds", Vol. XVI (1962) und Supplement I (1970)) oder durch Derivatisierung von Cyanurchlorid (vgl. z.B. "The Chemistry of Heterocyclic Compounds", L. Rapoport: "s-Triazines and Derivatives" (1959)).

Die Sulfonylisocyanate der Formel II sind ebenfalls zum großen Teil bekannt oder lassen sich nach im Prinzip bekannten Verfahren in einfacher Weise herstellen (vgl. DE-AS 1 211 165, DE-AS 1 226 565, DE-AS 1 230 016, DE-AS 1 568 640 sowie Chem. Ber. 105, 2791, 2800 (1972)).

Die Sulfonylcarbamoyl- bzw. -thiocarbamoylchloride der Formel III lassen sich nach üblichen Methoden aus den Alkalisalzen der entsprechenden Sulfonamide der Formel V, welche aus der Literatur bekannt sind, durch Umsetzung mit Phosgen oder Thiophosgen darstellen.

Die für die Umsetzungen nach b) benötigten Isothiocyanate der Formel VI sind bekannt oder nach bekannten Verfahren zugänglich (vgl. Tetrahedron 29, 691 (1973); Japan Kokai Sho-51-143686).

Gleiches gilt für die heterocyclischen Carbamoylchloride und Thiocarbamoylchloride der Formel VII (vgl. z.B. DE-AS 1 149 718 und DE-AS 2 238 870).

Sulfonylharnstoffe der allgemeinen Formel I, welche im aliphatischen Rest $R_1$ eines oder mehrere asymmetrische Kohlenstoffatome enthalten, liegen in enantiomeren oder diastereomeren Formen vor. Im allgemeinen werden die entsprechenden erfindungsgemäßen Verbindungen als Racemate oder als Diastereomerengemische erhalten. Falls erwünscht, können die üblichen Techniken zur Trennung dieser Gemische in die sterisch einheitlichen Bestandteile angewendet werden. Auch durch Verwendung von sterisch einheitlichen Ausgangsmaterialien ist eine Reindarstellung der genannten Verbindungen möglich. Werden z.B. Aminoheterocyclen der allgemeinen Formel IV mit threo- bzw. erythro-1,2-Dichlorpropyl-sulfonylisocyanat umgesetzt, so werden diesen entsprechende threo- bzw. erythro-1,2-Dichlorpropylsulfonylharnstoffe erhalten. Ferner können Sulfonylharnstoffe der allgemeinen Formel I, welche im aliphatischen Rest $R_1$ eine oder mehrere Doppelbindungen enthalten, bei entsprechender olefinischer Substitution als E- oder als Z-Isomere vorkommen, deren Reindarstellung bzw. Trennung ebenfalls möglich ist. Werden z.B. ungesättigte Sulfonylisocyanate der allgemeinen Formel II als E- oder als Z-Isomere eingesetzt, so werden die ungesättigten Sulfonylharnstoffe der Formel I in sterisch einheitlicher Form geliefert.

Die erfindungsgemäßen Verbindungen zeigen eine ausgezeichnete herbizide Wirkung und in wichtigen Großkulturen eine sehr gute Selektivität und eignen sich daher zur selektiven Bekämpfung zahlreicher zweikeimblättriger und grasartiger

annueller und perennierender Unkräuter in landwirtschaftlich bedeutenden Kulturen wie z.B. Weizen, Gerste, Roggen, Reis und Mais, Zuckerrüben, Soja und Baumwolle im Vorsaat-, Vorauflauf- und Nachauflaufverfahren.

Werden die erfindungsgemäßen Verbindungen vor dem Keimen der Unkrautpflanzen im Vorsaat- oder Vorauflaufverfahren auf die Erdoberfläche appliziert, so wird das Auflaufen der Keimlinge nicht verhindert. Die Unkräuter wachsen bis zum Keimblattstadium heran, stellen jedoch dann ihr Wachstum ein und sterben schließlich nach einigen Wochen vollkommen ab. Bei Applikation der Wirkstoffe auf die grünen Pflanzenteile im Nachauflaufverfahren tritt ebenfalls sehr rasch nach der Behandlung ein drastischer Wachstumsstop ein und die Unkrautpflanzen bleiben in dem zum Applikationszeitpunkt vorhandenen Wachstumsstadium stehen oder sterben nach einer gewissen Zeit ganz ab, so daß auf diese Weise eine für die Kulturpflanzen schädliche Unkrautkonkurrenz sehr früh und nachhaltig beseitigt werden kann.

Darüberhinaus weisen die erfindungsgemäßen Substanzen hervorragende wachstumsregulatorische Eigenschaften bei Kul-

./9

turpflanzen auf. Sie greifen regulierend in den pflanzeneigenen Stoffwechsel ein und können damit zur gezielten Beeinflussung von Pflanzeninhaltsstoffen und zur Ernteerleichterung wie z.B. durch Auslösen von Desikkation und Wuchsstauchung eingesetzt werden. Desweiteren eignen sie sich auch zur generellen Steuerung und Hemmung von unerwünschtem vegetativen Wachstum, ohne daß sie dabei die Pflanzen abtöten. Eine Hemmung des vegetativen Wachstums spielt bei vielen mono- und dikotylen Kulturen eine große Rolle, da das Lagern hierdurch verringert oder völlig verhindert werden kann.

Gegenstand der Erfindung sind daher auch herbizide bzw. wachstumsregulierende Mittel, die gekennzeichnet sind durch einen Behalt an einer Verbindung der Formel I in Kombination mit üblichen Formulierungshilfsmitteln und Inertstoffen sowie deren Verwendung im landwirtschaftlichen Bereich.

Die erfindungsgemäßen Mittel enthalten die Wirkstoffe der Formel I im allgemeinen zu 2 - 95 Gew.-%. Sie können als Spritzpulver, emulgierbare Konzentrate, versprühbare Lösungen, Stäubemittel oder Granulate in den üblichen Zubereitungen angewendet werden.

Spritzpulver sind in Wasser gleichmäßig dispergierbare Präparate, die neben dem Wirkstoff außer einem Verdünnungsmittel oder Inertstoff noch Netzmittel z.B. polyoxethylierte Alkylphenole, polyoxethylierte Fettalkohole, Alkyl- oder Alkylphenyl-sulfonate und Dispergiermittel, z.B. ligninsulfonsaures Natrium, 2,2'-dinaphthylmethan-6,6'-disulfonsaures Natrium, dibutylnaphthalinsulfonsaures Natrium oder auch oleylmethyltaurinsaures Natrium enthalten.

Emulgierbare Konzentrate werden durch Auflösen des Wirkstoffes in einem organischen Lösungsmittel, z.B. Butanol, Cyclohexanon, Dimethylformamid, Xylol oder auch höhersiedenden Aromaten oder Kohlenwasserstoffen unter Zusatz von einem oder mehreren Emulgatoren hergestellt. Als Emulgatoren kön-

nen beispielsweise verwandt werden: Alkylarylsulfonsaure Kalziumsalze wie Ca-dodecylbenzosulfonat oder nichtionische Emulgatoren wie fettsäurepolyglykolester, Alkylarylpolyglykolether, Fettalkoholpolyglykolether, Propylenoxid-Ethylenoxid-Kondensationsprodukte, Fettalkohol-Propylenoxid-Ethylenoxid-Kondensationsprodukte, Alkylpolyether, Sorbitanfettsäureester, Polyoxethylensorbitan-fettsäureester oder Polyoxethylen-sorbitester.

Stäubemittel erhält man durch Vermahlen des Wirkstoffes mit fein verteilten, festen Stoffen, z.B. Talkum, natürlichen Tonen, wie Kaolin, Bentonit, Pyrophillit oder Diatomeenerde.

Granulate können entweder durch Verdüsen des Wirkstoffes auf adsorptionsfähiges, granuliertes Inertmaterial hergestellt werden oder durch Aufbringen von Wirkstoffkonzentraten mittels Klebemitteln z.B. Polyvinylalkohol, polyacrylsaurem Natrium oder auch Mineralölen auf die Oberfläche von Trägerstoffen wie Sand, Kaolinite oder von granuliertem Inertmaterial. Auch können geeignete Wirkstoffe in der für die Herstellung von Düngemittelgranalien üblichen Weise - gewünschtenfalls in Mischung mit Düngemitteln - hergestellt werden.

Bei herbiziden Mitteln können die Konzentrationen der Wirkstoffe in den handelsüblichen Formulierungen verschieden sein.

In Spritzpulvern variiert die Wirkstoffkonzentration z.B. zwischen etwa 10 % und 80 %, der Rest besteht aus den oben angegebenen Formulierungszusätzen. Bei emulgierbaren Konzentraten kann die Wirkstoffkonzentration gleichfalls etwa 10 % bis 80 % betragen. Staubförmige Formulierungen enthalten etwa 2 - 20 %. Bei Granulaten hängt der Wirkstoffgehalt zum Teil davon ab, ob die wirksame Verbindung flüssig oder fest vorliegt und welche Granulierhilfsmittel, Füllstoffe usw. verwendet werden.

Zur Anwendung als Herbizide werden die handelsüblichen Konzentrate gegebenenfalls in üblicher Weise verdünnt, z.B. bei Spritzpulvern und emulgierbaren Konzentraten mittels Wasser. Staubförmige und granulierte Zubereitungen sowie versprühbare Lösungen werden vor der Anwendung nicht mehr mit weiteren inerten Stoffen verdünnt. Mit den äußeren Bedingungen wie Temperatur, Feuchtigkeit, u.a. variiert die erforderliche Aufwandmenge. Sie beträgt im allgemeinen zwischen 0.01 und 10 kg/ha, vorzugsweise etwa 0.1 bis 5.0 kg/ha Wirkstoff.

Für einige Anwendungsgebiete kann es zweckmäßig sein, die neuen Herbizide mit einem oder mehreren Herbiziden gemeinsam anzuwenden, z.B. als "Tankmischung" oder in Form einer Fertigformulierung, um weitere vorteilhafte Wirkungen zu erzielen.

Die erfindungsgemäßen Wirkstoffe können mit anderen Herbiziden, Insektiziden und Fungiziden kombiniert werden.

Zur Anwendung als Wachstumsregulatoren eignen sich Konzentrationen zwischen 0.01 und 1.25 kg/ha. Bevorzugt verwendet man wäßrige Dispersionen von Spritzpulvern oder Verdünnungen von emulgierbaren Konzentraten. Die Anwendung erfolgt im Nachauflauf. Bevorzugte Kulturen sind Mais und Tabak.

## Herstellungsbeispiele

### Beispiel 1

**N-(4-Methoxy-6-methyl-1,3,5-triazin-2-yl)-N'-(2,2,2-trichlor-eth-1-oxysulfonyl)-harnstoff**

14,0 g(0,1 mol) 2-Amino-4-methoxy-6-methyl-1,3,5-triazin werden in 250 ml Dichlormethan suspendiert und bei 0 bis 5°C tropfenweise mit einer Lösung von 25,5 g (0,1 mol) 2,2,2-Trichlor-eth-1-oxy-sulfonylisocycanat in 100 ml Dichlormethan versetzt. Man rührt 18 Stunden bei Raumtemperatur nach, kühlt das Reaktionsgemisch auf 0 °C und versetzt mit n-Hexan. Das ausgefallene Reaktionsprodukt wird abgesaugt und aus abs. Ethanol umkristallisiert. Man erhält 35,3 g (89,4 % d.Th.) an N-(4-Methoxy-6-methyl-1,3,5-triazin-2-yl)-N'-(2,2,2-trichlor-eth-1-oxy-sulfonyl)-harnstoff vom Schmelzpunkt 128 °C.

### Beispiel 2

**N-(4,6-Dimethoxy-1,3,5-triazin-2-yl)-N'-(2-chlor-n-prop-1-yl-sulfonyl)-harnstoff**

15,6 g (0,1 mol) 2-Amino-4,6-dimethoxy-1,3,5-triazin werden in 150 ml Dichlormethan suspendiert und bei 0 °C unter Rühren mit einer Lösung von 19,5 g (0,106 mol) 2-Chlor-n-prop-1-yl-sulfonylisocyanat in 100 ml Dichlormethan versetzt. Man rührt 12 Stunden bei Raumtemperatur nach, kühlt auf 0 °C und versetzt mit 300 ml n-Hexan. Das ausgefallene Reaktionsprodukt wird

abgesaugt, mit n-Hexan gewaschen und getrocknet. Man erhält 30,7 g (90,5 % d.Th.) an N-(4,6-Dimethoxy-1,3,5-triazin-2-yl)-N'-(2-chlor-n-prop-1-yl-sulfonyl)-harnstoff vom Schmelzpunkt 139-41 °C.

Beispiel 3

N-(4,6-Dimethyl-5-chlor-pyrimidin-2-yl)-N'-(1,2-dichlor-n-prop-1-yl-sulfonyl)-harnstoff

15,3 g (0,05 mol) N-(4,6-Dimethyl-5-chlorpyrimidin-2-yl)-N'-(n-prop-1-en-1-yl-sulfonyl)-harnstoff (vgl. Beispiel 23) werden in 300 ml Dichlormethan suspendiert und unter UV-Bestrahlung 30 min chloriert. Man saugt das ausgefallene Reaktionsprodukt ab und wäscht mit n-Hexan. Nach Umkristallisieren aus Chloroform erhält man 14,6 g (78 % d.Th.) an N-(4,6-Dimethyl-5-chlor-pyrimidin-2-yl)-N'-(1,2-dichlor-n-prop-1-yl-sulfonyl)-harnstoff vom Schmelzpunkt 161 - 2 °C.

Beispiel 4

N-(4,6-Dimethyl-pyrimidin-2-yl)-N'-(vinylsulfonyl)-harnstoff

14,6 g (0,05 mol) N-(4,6-Dimethyl-pyrimidin-2-yl)-N'-(2-chlor-eth-1-yl-sulfonyl)-harnstoff (vgl. Beispiel 12) werden in 150 ml Ethanol gelöst und bei Raumtemperatur

BAD ORIGINAL

mit 4 g (0,1 Mol) Natriumhydroxid-gelöst in 10 ml Wasser- versetzt. Anschließend wird das Reaktionsgemisch 4 Stunden auf 40 °C erwärmt, im Vakuum eingeengt und in 150 ml Wasser aufgenommen. Nach dem Ansäuern mit 2 n HCl (pH 5) wird mit Essigester extrahiert, anschließend werden die Extrakte getrocknet und eingeengt. Nach Zugabe von n-Hexan erhält man 8,9 g (69,5 % d.Th.) an N-(4,6-Dimethylpyrimidin-2-yl)-N'-(vinylsulfonyl)-harnstoff vom Schmelzpunkt 131-3°C.

BAD ORIGINAL

Tabelle 1

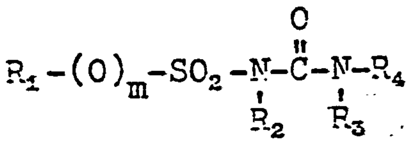

| Bei-spiel Nr. | $R_1$ | m | $R_2$ | $R_3$ | $R_4$ | Schmp. [°C] |
|---|---|---|---|---|---|---|
| 5 | $CCl_3CH_2-$ | 1 | H | H | 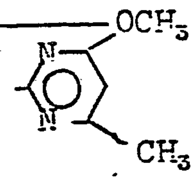 | 155-60 |
| 6 | $CF_3CH_2-$ | 1 | H | H | 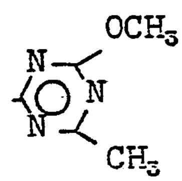 | 128-33 |
| 7 | $CF_3CH_2-$ | 1 | H | H | 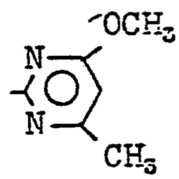 | 120-30 |
| 8 | 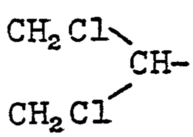 | 1 | H | H | 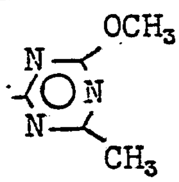 | 132-6 |
| 9 | 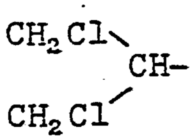 | 1 | H | H | 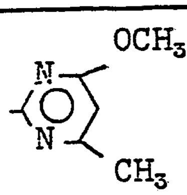 | 127-133 |
| 10 | 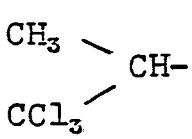 | 1 | H | H | 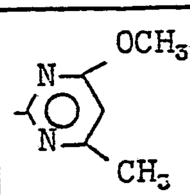 | 143-6 |
| 11 | 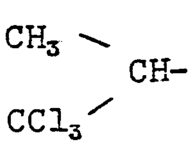 | 1 | H | H | 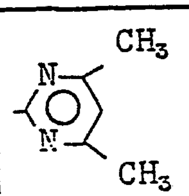 | |

BAD ORIGINAL

Tabelle 1 (Fortsetzung)

| Beispiel Nr. | $R_1$ | m | $R_2$ | $R_3$ | $R_4$ | Schmp. [°C] |
|---|---|---|---|---|---|---|
| 12 | $CH_2=CH-CH_2-$ | 1 | H | H | 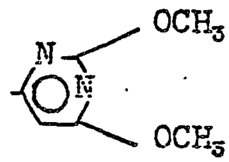 | 80-2 |
| 13 | 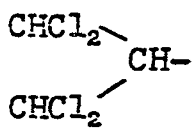 | 1 | H | H | 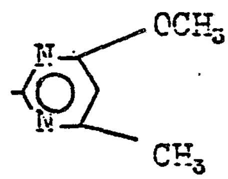 | |
| 14 | 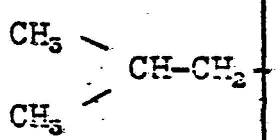 | 1 | H | H | 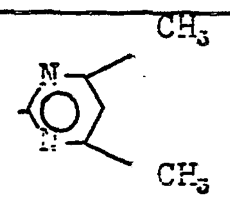 | 140-3 |
| 15 | 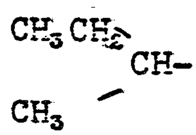 | 1 | H | H | 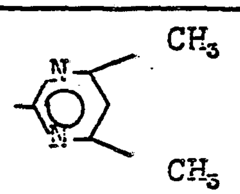 | |
| 16 | 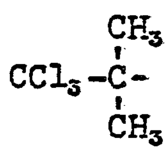 | 1 | H | H | 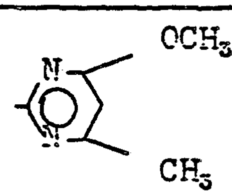 | |
| 17 | 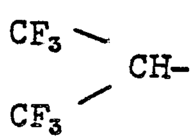 | 1 | H | H | 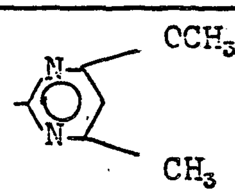 | |
| 18 | $BrCH_2CHBrCH_2-$ | 1 | H | H | 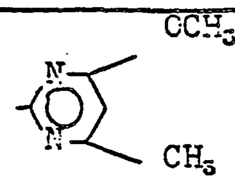 | |
| 19 | $ClCH_2CH_2-$ | 0 | H | H | 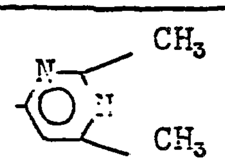 | 95-6 |

BAD ORIGINAL

Tabelle 1 (Fortsetzung)

| Bei-spiel Nr. | $R_1$ | m | $R_2$ | $R_3$ | $R_4$ | Schmp. [°C] |
|---|---|---|---|---|---|---|
| 20 | $ClCH_2CH_2-$ | 0 | H | H | 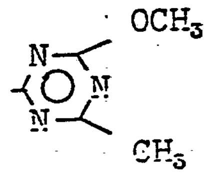 | 159-60 |
| 21 | $ClCH_2CH_2-$ | 0 | H | H | 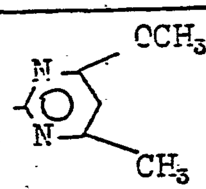 | 170 |
| 22 | $ClCH_2CH_2-$ | 0 | H | H | 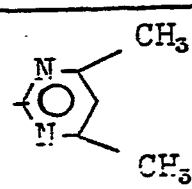 | 165-7 |
| 23 | $Cl_2CHCH_2-$ | 0 | H | H | 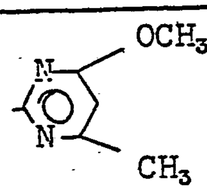 | 142-3 |
| 24 | $Cl_2CHCH_2-$ | 0 | H | H | 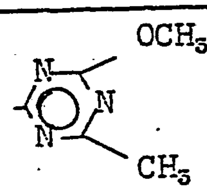 | 133-5 |
| 25 | $ClCH_2CHCl-$ | 0 | H | H | 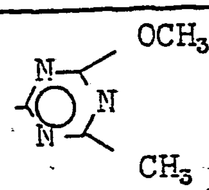 | 110-2 |
| 26 | $BrCH_2CHBr-$ | 0 | H | H | 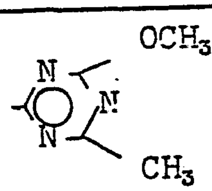 | |

BAD ORIGINAL

Tabelle 1 (Fortsetzung)

| Beispiel Nr. | $R_1$ | m | $R_2$ | $R_3$ | $R_4$ | Schmp. [°C] |
|---|---|---|---|---|---|---|
| 27 | $CH_2=CH-$ | 0 | H | H | 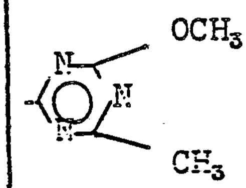 | 147-50 |
| 28 | $CH_2=CH-$ | 0 | H | H | 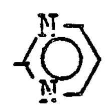 | 183-5 |
| 29 | $ClCH=CH-$ | 0 | H | H | 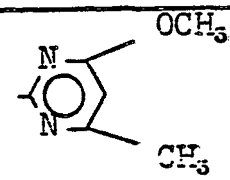 | |
| 30 | $CH_2=C(Br)-$ | 0 | H | H | 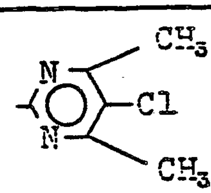 | |
| 31 | $CH_3CH(Cl)CH_2-$ | 0 | H | H | 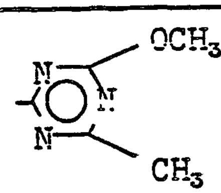 | 116-8 |
| 32 | $CH_3-CH(Cl)CH_2-$ | 0 | H | H | 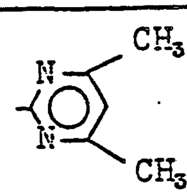 | 165-7 |
| 33 | $CH_3CH(Cl)CH_2-$ | 0 | H | H | 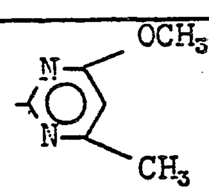 | 137-42 |

BAD ORIGINAL

Tabelle 1 (Fortsetzung)

| Beispiel | $R_1$ | m | $R_2$ | $R_3$ | $R_4$ | Schmp. [°C] |
|---|---|---|---|---|---|---|
| 34 | $CH_3CH(Cl)CH_2-$ | 0 | H | H | 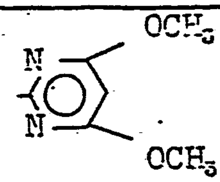 | 165-7 |
| 35 | $CH_3CH(Cl)CH_2-$ | 0 | H | H | 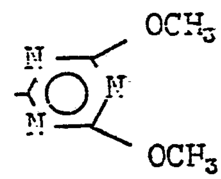 | 139-41 |
| 36 | $CH_3CH(Cl)CH_2-$ | 0 | H | H | 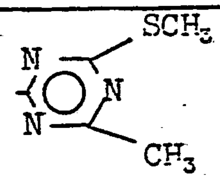 | 158-60 |
| 37 | $CH_3CH(Cl)CH_2-$ | 0 | H | H | 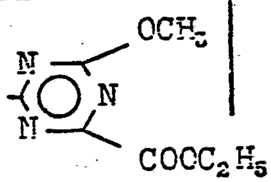 | 89 |
| 38 | $CH_3CH(Cl)CH_2-$ | 0 | H | H | 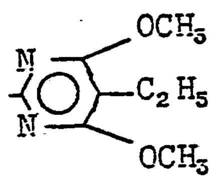 | 174-6 |
| 39 | $CH_3CH(Cl)CH_2-$ | 0 | H | H | 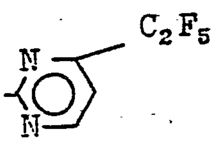 | 153-4 |
| 40 | $CH_3CH(Cl)CH_2-$ | 0 | H | H | 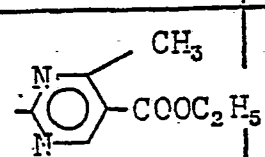 | 149-52 |

BAD ORIGINAL

Tabelle 1 (Fortsetzung)

| Beispiel Nr. | $R_1$ | m | $R_2$ | $R_3$ | $R_4$ | Schmp. [°C] |
|---|---|---|---|---|---|---|
| 41 | $CH_3CHCH_2-$<br>Cl | 0 | H | H | 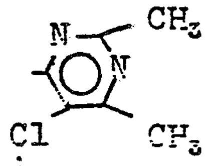 | 149 |
| 42 | $CH_3CH=CH-$ | 0 | H | H | 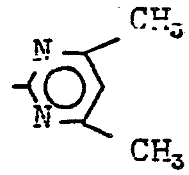 | 158-61 |
| 43 | $CH_3CH=CH-$ | 0 | H | H | 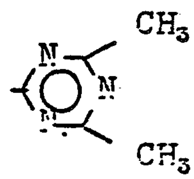 | 154-8 |
| 44 | $CH_3CH=CH-$ | 0 | H | H | 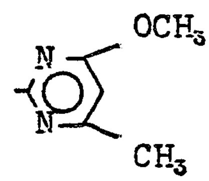 | 215-9 |
| 45 | $CH_3CH=CH-$ | 0 | H | H | 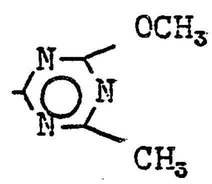 | 172-3 |
| 46 | $CH_3CH=CH-$ | 0 | H | H | 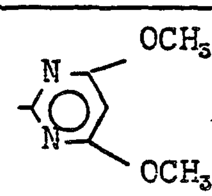 | 202-3 |
| 47 | $CH_3CH=CH-$ | 0 | H | H | 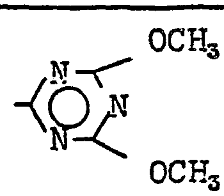 | 165-8 |

BAD ORIGINAL

Tabelle 1 (Fortsetzung)

| Beispiel Nr. | $R_1$ | m | $R_2$ | $R_3$ | $R_4$ | Schmp. [°C] |
|---|---|---|---|---|---|---|
| 48 | $CH_3CH=CH-$ | 0 | H | H | 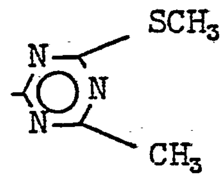 | 176-8 |
| 49 | $CH_3CH=CH-$ | 0 | H | H | 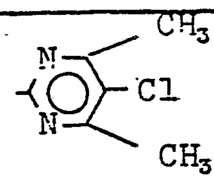 | 111 |
| 50 | $CH_3CH=CH-$ | 0 | H | H | 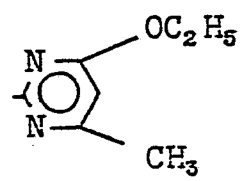 | 204-6 |
| 51 | $CH_3CH=CH-$ | 0 | H | H | 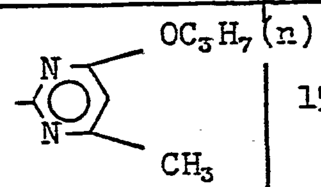 | 159-61 |
| 52 | $CH_3CH=CH-$ | 0 | H | H | 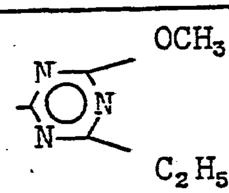 | 142-4 |
| 53 | $CH_3CH=CH-$ | 0 | H | H | 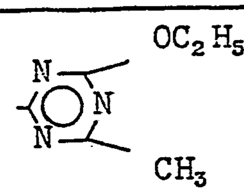 | 154-7 |
| 54 | $CH_3CH=CH-$ | 0 | H | H | 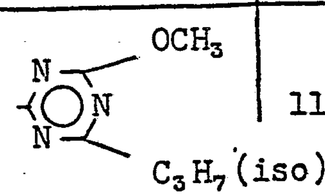 | 114-6 |

Tabelle 1 (Fortsetzung)

| Beispiel Nr. | $R_1$ | | m | $R_2$ | $R_3$ | $R_4$ | Schmp. [°C] |
|---|---|---|---|---|---|---|---|
| 55 | $CH_3\underset{Cl}{CH}\underset{Cl}{CH}-$ | (THREO) | 0 | H | H | 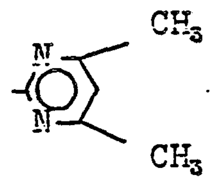 | 110-2 |
| 56 | $CH_3\underset{Cl}{CH}\underset{Cl}{CH}-$ | ( " ) | 0 | H | H | 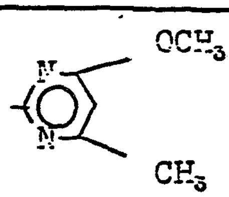 | 166-7 |
| 57 | $CH_3\underset{Cl}{CH}\underset{Cl}{CH}-$ | ( " ) | 0 | H | H | 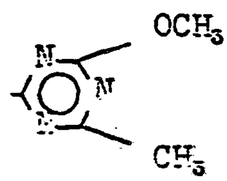 | 130 |
| 58 | $CH_3\underset{Cl}{CH}\underset{Cl}{CH}-$ | ( " ) | 0 | H | H | 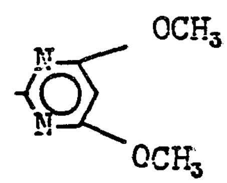 | 170-2 |
| 59 | $CH_3\underset{Cl}{CH}\underset{Cl}{CH}-$ | ( " ) | 0 | H | H | 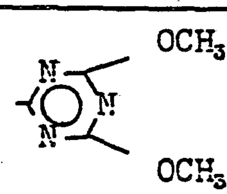 | 139-42 |
| 60 | $CH_3\underset{Cl}{CH}\underset{Cl}{CH}$ | ( " ) | 0 | H | $CH_3$ | 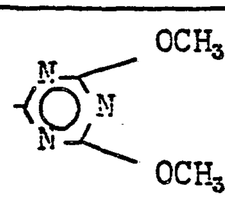 | 101-2 |
| 61 | $CH_3\underset{Cl}{CH}\underset{Cl}{CH}-$ | ( " ) | 0 | H | H | 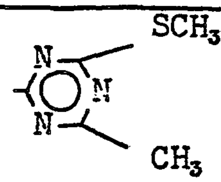 | 124-6 |

BAD ORIGINAL

Tabelle 1 (Fortsetzung)

| Beispiel Nr. | $R_1$ | m | $R_2$ | $R_3$ | $R_4$ | Schmp. [°C] |
|---|---|---|---|---|---|---|
| 62 | $CH_3CHCH-$<br>Cl Cl (THREO) | 0 | H | H | 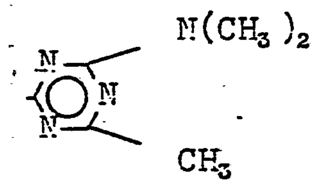 | 141-3 |
| 63 | $CH_3CHCH-$<br>Cl Cl ( " ) | 0 | H | H | 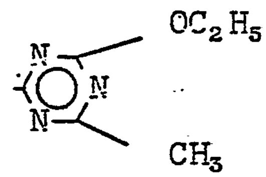 | 98-102 |
| 64 | $CH_3CHCH-$<br>Cl Cl ( " ) | 0 | H | H | 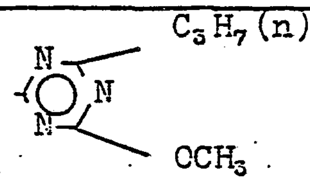 | Harz |
| 65 | $CH_3CHCH-$<br>Cl Cl ( " ) | 0 | H | H | 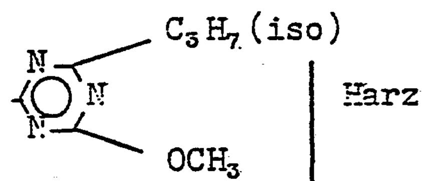 | Harz |
| 66 | $CH_3CHCH-$<br>Cl Cl ( " ) | 0 | H | H | 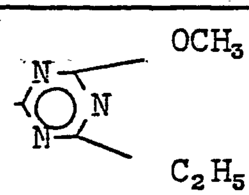 | 50-3 |
| 67 | $CH_3CHCH-$<br>Cl Cl ( " ) | 0 | H | H | 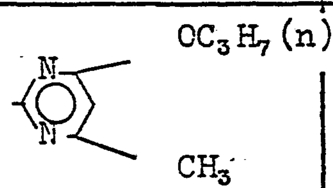 | 112-4 |
| 68 | $CH_3CHCH-$<br>Cl Cl (ERYTHRO) | 0 | H | H | 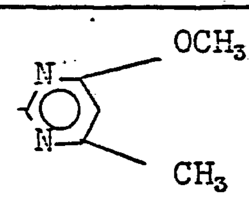 | 124-30 |

BAD ORIGINAL

Tabelle 1 (Fortsetzung)

| Beispiel Nr. | $R_1$ | m | $R_2$ | $R_3$ | $R_4$ | Schmp. [°C] |
|---|---|---|---|---|---|---|
| 69 | 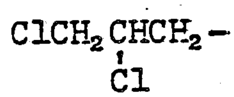 | 0 | H | H | 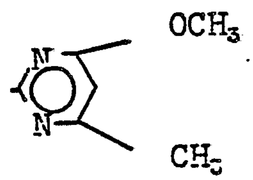 | 131-4 |
| 70 | 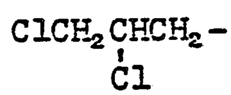 | 0 | H | H | 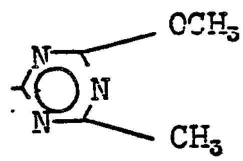 | 128-30 |
| 71 | 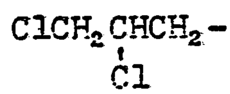 | 0 | H | H | 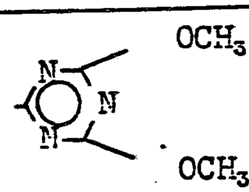 | 111-3 |
| 72 | 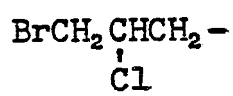 | 0 | H | H | 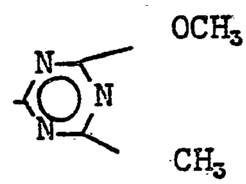 | |
| 73 | 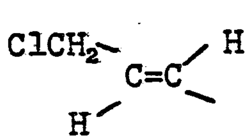 | 0 | H | H | 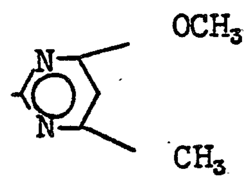 | 163-7 |
| 74 | 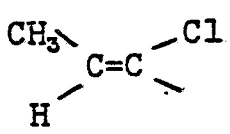 | 0 | H | H | 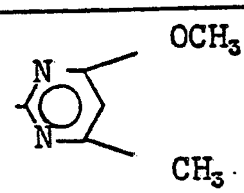 | 151-3 |
| 75 | 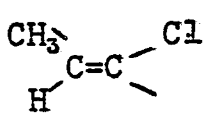 | 0 | H | H | 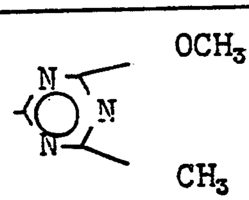 | 95 |

Tabelle 1 (Fortsetzung)

| Beispiel Nr. | $R_1$ | m | $R_2$ | $R_3$ | $R_4$ | Schmp. [°C] |
|---|---|---|---|---|---|---|
| 76 | 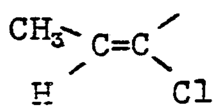 | 0 | H | H | 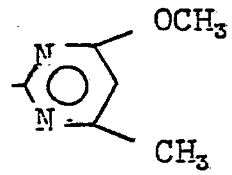 | 164-7 |
| 77 | 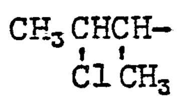 | 0 | H | H | 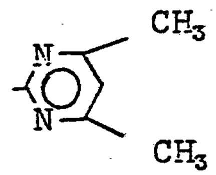 | 112-4 |
| 78 | 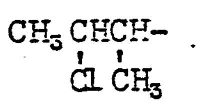 | 0 | H | H | 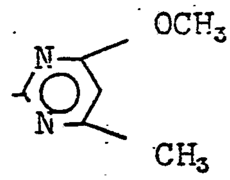 | 139-40 |
| 79 | 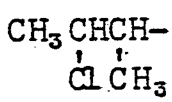 | 0 | H | H | 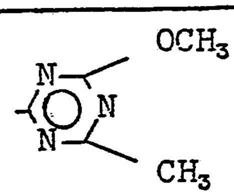 | 136-8 |
| 80 | 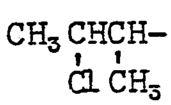 | 0 | H | H | 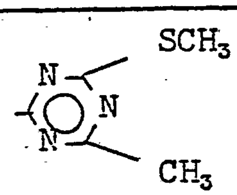 | 158-60 |
| 81 | 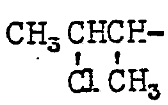 | 0 | H | H | 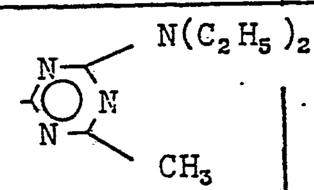 | 108-10 |
| 82 | 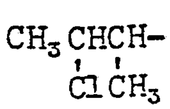 | 0 | H | H | 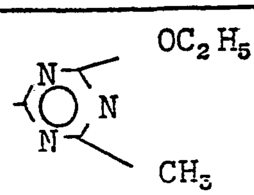 | 99-102 |

BAD ORIGINAL

Tabelle 1 (Fortsetzung)

| Beispiel Nr. | $R_1$ | m | $R_2$ | $R_3$ | $R_4$ | Schmp. [°C] |
|---|---|---|---|---|---|---|
| 83 | $CH_3CHCH-$<br>$Cl\ CH_3$ | 0 | H | H | 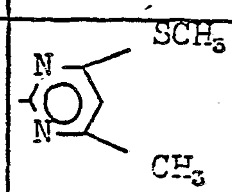 | 116-20 |
| 84 | $CH_3CHCH-$<br>$ClCH_3$ | 0 | H | H | 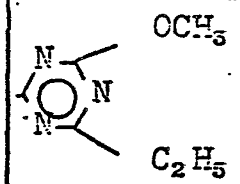 | 121-3 |
| 85 | $CH_3CHCH-$<br>$Cl\ CH_3$ | 0 | H | H | 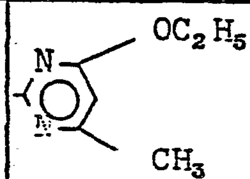 | 116-8 |
| 86 | $CH_3CH_2CHCH_2-$<br>$Cl$ | 0 | H | H | 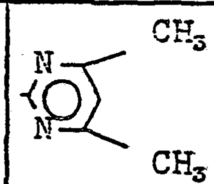 | 120-1 |
| 87 | $CH_3CH_2CHCH_2-$<br>$Cl$ | 0 | H | H | 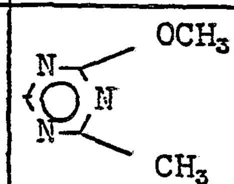 | Harz |
| 88 | $CH_3$<br>$CH_3-CCH_2-$<br>$Cl$ | 0 | H | H | 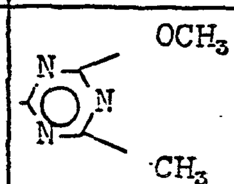 | Harz |
| 89 | $CH_3$<br>$CH_3-C-CH_2-$<br>$Cl$ | 0 | H | H | 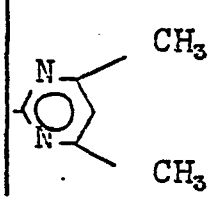 | Harz |

BAD ORIGINAL

Tabelle 1 (Fortsetzung)

| Bei-spiel Nr. | $R_1$ | m | $R_2$ | $R_3$ | $R_4$ | Schmp. [°C] |
|---|---|---|---|---|---|---|
| 90 | $CH_3CHCHCH_2-$<br>Cl Cl | 0 | H | H | $CH_3$ 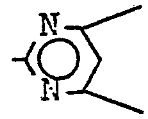 $CH_3$ | Harz |
| 91 | $CH_3CHCHCH_2-$<br>Cl Cl | 0 | H | H | $OCH_3$ 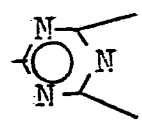 $CH_3$ | Harz |
| 92 | $CH_3$<br>$ClCH_2C-CH_2-$<br>Cl | 0 | H | H | $CH_3$ 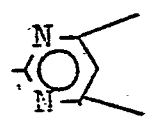 $CH_3$ | 164-6 |
| 93 | $CH_3$<br>$ClCH_2CCH_2-$<br>Cl | 0 | H | H | $OCH_3$ 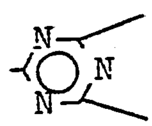 $CH_3$ | 131-4 |
| 94 | $CHCl_2CH_2CHCH_2-$<br>Cl | 0 | H | H | $OCH_3$ 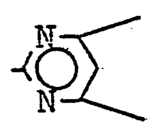 $CH_3$ | 105 |
| 95 | $CHCl_2CH_2CHCH_2-$<br>Cl | 0 | H | H | $OCH_3$ 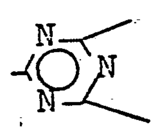 $CH_3$ | 120 |
| 96 | $CHCl_2CH_2CHCH-$<br>Br Br | 0 | H | H | $OCH_3$ 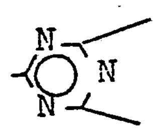 $CH_3$ | Harz |

BAD ORIGINAL

Tabelle 1 (Fortsetzung)

| Beispiel Nr. | $R_1$ | m | $R_2$ | $R_3$ | $R_4$ | Schmp. [°C] |
|---|---|---|---|---|---|---|
| 97 | $CHCl_2CH_2CHCH-$<br>Br Br | 0 | H | H | 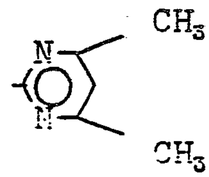 | 85-90 |
| 98 | $CH_3CHCH-$<br>$ClCH_2Cl$ | 0 | H | H | 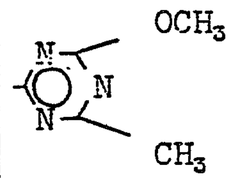 | Harz |
| 99 | $CH_3CHCH-$<br>$ClCH_2Cl$ | 0 | H | H | 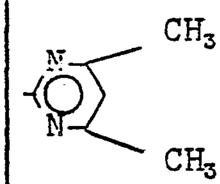 | Harz |
| 100 | CHClBrCHBrCHBrCHBr- | 0 | H | H | 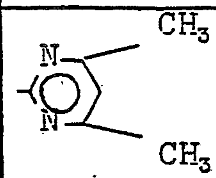 | 140-4 |
| 101 | CHClBrCHBrCHBrCHBr- | 0 | H | H | 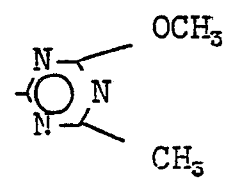 | |
| 102 | $CH_3OCH_2CHCH_2-$<br>Cl | 0 | H | H | 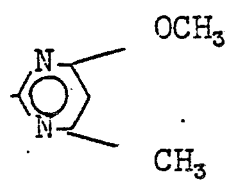 | 144-6 |
| 103 | $CH_3OCH_2CHCH_2-$<br>Cl | 0 | H | H | 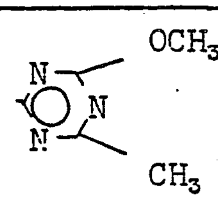 | 117-20 |

BAD ORIGINAL

Tabelle 1 (Fortsetzung)

| Beispiel Nr. | $R_1$ | m | $R_2$ | $R_3$ | $R_4$ | Schmp. [°C] |
|---|---|---|---|---|---|---|
| 104 | $CH_3CH_2CH=CH-$ | 0 | H | H | 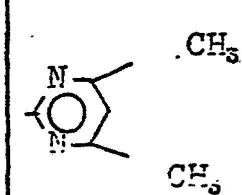 | 143-6 |
| 105 | $CH_3CH_2CH=CH-$ | 0 | H | H | 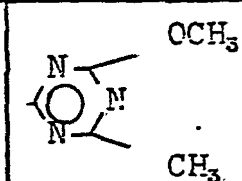 | 136-9 |
| 106 | $CH_3CH=\underset{CH_3}{C}-$ +) | 0 | H | H | 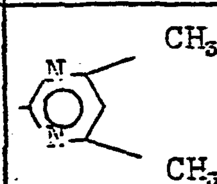 | 167-71 |
| 107 | $CH_3CH=\underset{CH_3}{C}-$ +) | 0 | H | H | 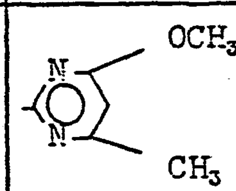 | 148-54 |
| 108 | $CH_3CH=\underset{CH_3}{C}-$ +) | 0 | H | H | 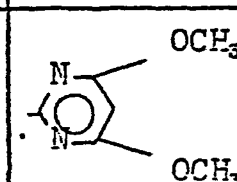 | 143-6 |
| 109 | $CH_3CH=\underset{CH_3}{C}-$ +) | 0 | H | H | 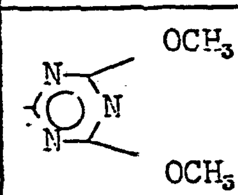 | 158 |
| 110 | $CH_3CH=\underset{CH_3}{C}-$ +) | 0 | H | $CH_3$ | 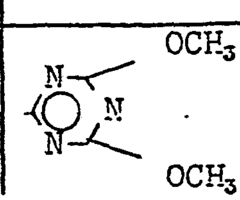 | 121-123 |

+) E/Z-Gemisch

BAD ORIGINAL

Tabelle 1 (Fortsetzung)

| Beispiel Nr. | $R_1$ | m | $R_2$ | $R_3$ | $R_4$ | Schmp. [°C] |
|---|---|---|---|---|---|---|
| 111 | $CH_3CH=C-$ +)<br>$CH_3$ | 0 | H | H | 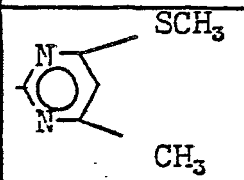 | 153-6 |
| 112 | $CH_3CH=C-$ +)<br>$CH_3$ | 0 | H | H | 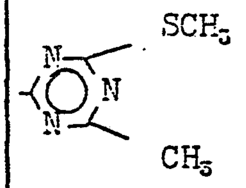 | 156-8 |
| 113 | 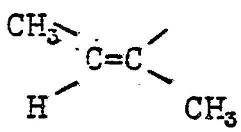 | 0 | H | H | 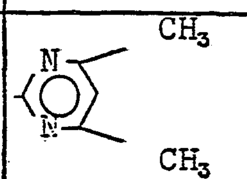 | 174-6 |
| 114 | 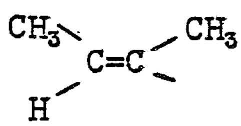 | 0 | H | H | 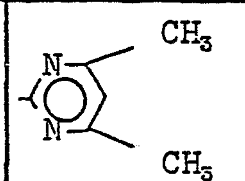 | 170-2 |
| 115 | 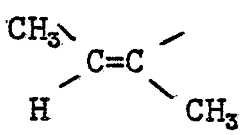 | 0 | H | H | 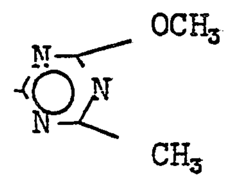 | 148-9 |
| 116 | 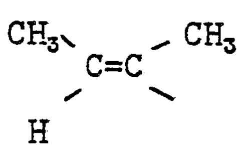 | 0 | H | H | 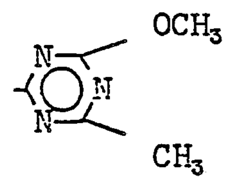 | 151-2 |
| 117 | 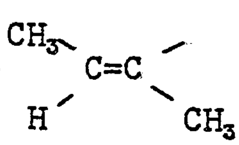 | 0 | H | H | 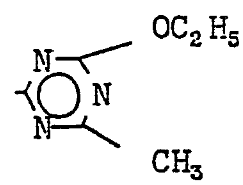 | 128-31 |

+) E/Z-Gemisch

BAD ORIGINAL

Tabelle 1 (Fortsetzung)

| Beispiel Nr. | $R_1$ | m | $R_2$ | $R_3$ | $R_4$ | Schmp. [°C] |
|---|---|---|---|---|---|---|
| 118 | 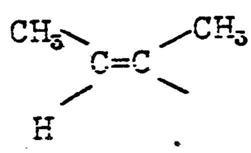 | 0 | H | H | 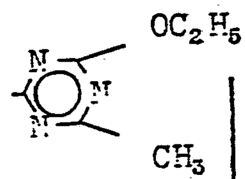 | 157-60 |
| 119 | 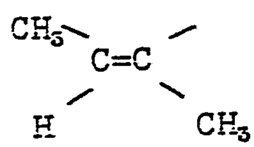 | 0 | H | H | 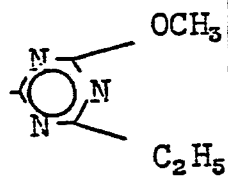 | 130-2 |
| 120 | 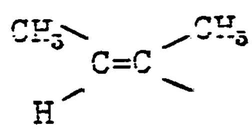 | 0 | H | H | 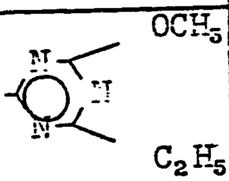 | 140-2 |
| 121 | $CHCl_2CH_2$-CH=CH- | 0 | H | H | 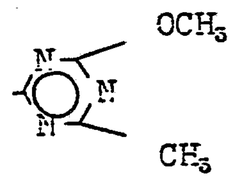 | Harz |
| 122 | $CHCl_2CH_2$CH=CH- | 0 | H | H | 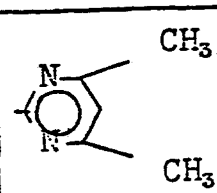 | Harz |
| 123 | $CH_3$CHClCH=CH- | 0 | H | H | 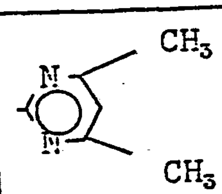 | 169-173 |
| 124 | 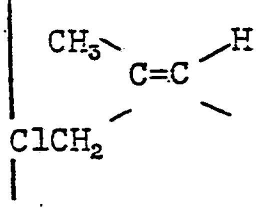 | 0 | H | H | 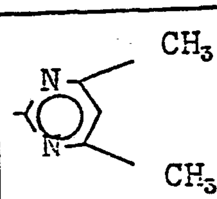 | Harz |

Tabelle 1 (Fortsetzung)

| Bei-spiel Nr. | $R_1$ | m | $R_2$ | $R_3$ | $R_4$ | Schmp. [°C] |
|---|---|---|---|---|---|---|
| 125 | 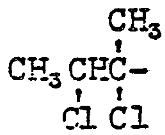 | 0 | H | H | 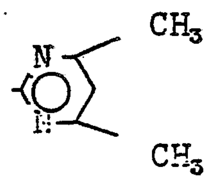 | |
| 126 | 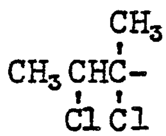 | 0 | H | H | 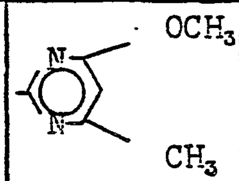 | |
| 127 | 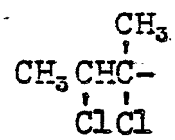 | 0 | H | H | 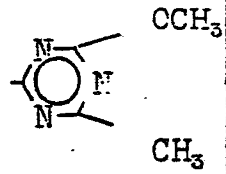 | Harz |
| 128 | 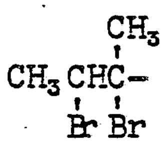 | 0 | H | H | 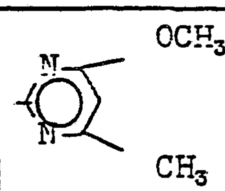 | 142-4 |
| 129 | 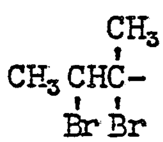 | 0 | H | H | 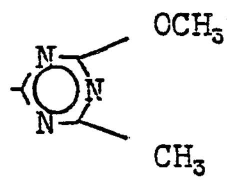 | 58-60 |
| 130 | 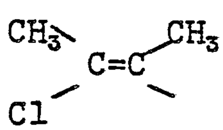 | 0 | H | H | 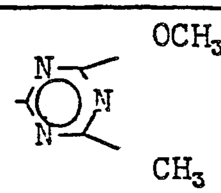 | Harz |
| 131 | 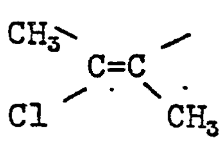 | 0 | H | H | 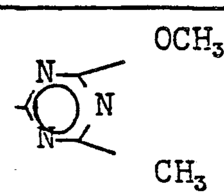 | |

Tabelle 1 (Fortsetzung)

| Bei-spiel Nr. | $R_1$ | n | $R_2$ | $R_3$ | $R_4$ | Schmp. [°C] |
|---|---|---|---|---|---|---|
| 132 | 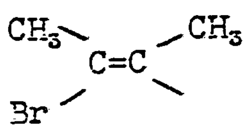 | 0 | H | H | 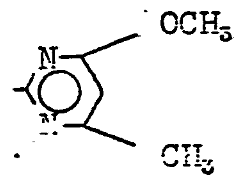 | |
| 133 | 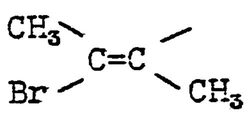 | 0 | H | H | 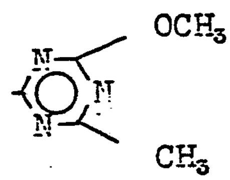 | |
| 134 | ClCH=CH-CH=CH- | 0 | H | H | 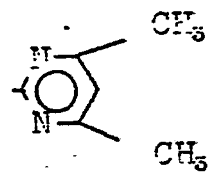 | 130-3 |
| 135 | $Cl_2CH-CH=CH-$ | 0 | H | $CH_3$ | 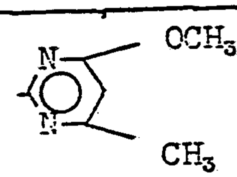 | 144-8 |
| 136 | $CH_3(CH_2)CHCH_2-$ (Cl an CH) | 0 | H | H | 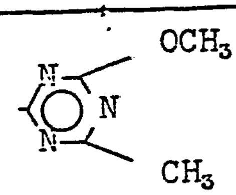 | Harz |
| 137 | $CH_3(CH_2)_2CHCH_2-$ (Cl an CH) | 0 | H | H | 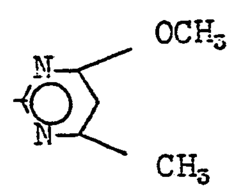 | 167 |
| 138 | $CH_3CH_2CHCH-$ (Cl, $CH_3$) | 0 | H | H | 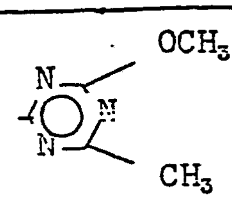 | 124-7 |

0061661

Tabelle 1 (Fortsetzung)

| Beispiel Nr. | $R_1$ | m | $R_2$ | $R_3$ | $R_4$ | Schmp. [°C] |
|---|---|---|---|---|---|---|
| 139 | 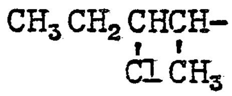 | 0 | H | H | 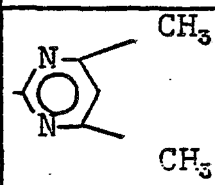 | Harz |
| 140 | $CH_3(CH_2)_2CH{=}CH-$ | 0 | H | H | 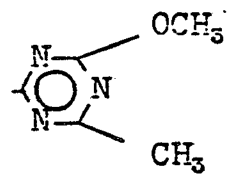 | |
| 141 | $CH_3(CH_2)_2CH{=}CH-$ | 0 | H | H | 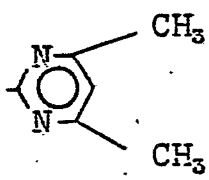 | |
| 142 | $CH_3(CH_2)_2CH{=}CH-$ | 0 | H | H | 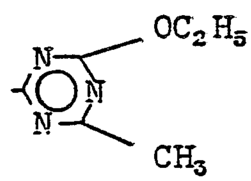 | |
| 143 | $CH_3(CH_2)_2CH{=}CH-$ | 0 | H | H | 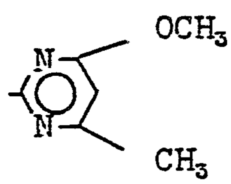 | 180-3 |
| 144 | 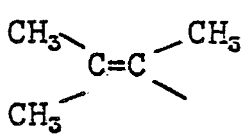 | 0 | H | H | 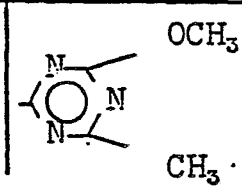 | Harz |

BAD ORIGINAL

Tabelle 1 (Fortsetzung)

| Beispiel Nr. | $R_1$ | m | $R_2$ | $R_3$ | $R_4$ | Schmp. [°C] |
|---|---|---|---|---|---|---|
| 145 | $CH_3(CH_2)_3CHCH_2-$ (Cl an CH) | 0 | H | H | 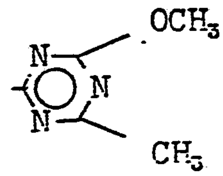 | Oel |
| 146 | $CH_3(CH_2)_3CHCH_2-$ (Cl an CH) | 0 | H | H | 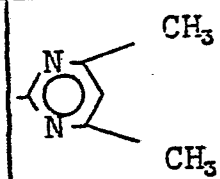 | |
| 147 | $CH_3(CH_2)_3CHCH_2-$ (Cl an CH) | 0 | H | H | 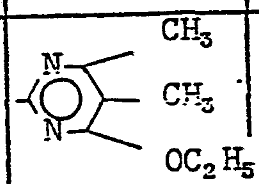 | 72-3 |
| 148 | $CH_3(CH_2)_3CH=CH-$ | 0 | H | H | 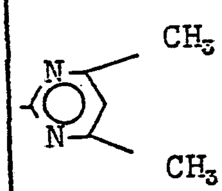 | 142-3 |
| 149 | $CH_3(CH_2)_3CH=CH-$ | 0 | H | H | 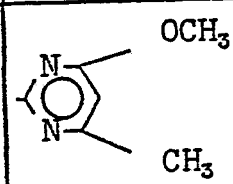 | 153-7 |
| 150 | $CH_3(CH_2)_3CH=CH-$ | 0 | H | H | 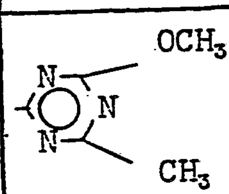 | 144 |
| 151 | $CH_3(CH_2)_3CH=CH-$ | 0 | H | H | 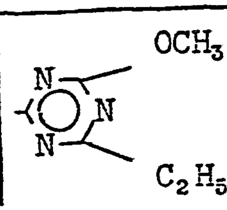 | 116-9 |

Tabelle 1 (Fortsetzung)

| Beispiel Nr. | $R_1$ | m | $R_2$ | $R_3$ | $R_4$ | Schmp. [°C] |
|---|---|---|---|---|---|---|
| 152 | $CH_3(CH_2)_4CH(Cl)CH_2-$ | 0 | H | H | 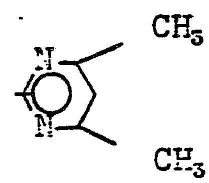 | |
| 153 | $CH_3(CH_2)_4CH(Cl)CH_2-$ | 0 | H | H | 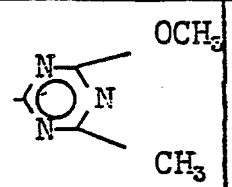 | |
| 154 | $CH_3(CH_2)_4CH{=}CH-$ | 0 | H | H | 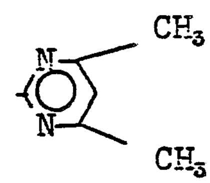 | 125-6 |
| 155 | $CH_3(CH_2)_5CH(Cl)CH_2-$ | 0 | H | H | 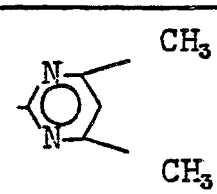 | 69-70 |
| 156 | $CH_3(CH_2)_5CH(Cl)CH_2-$ | 0 | H | H | 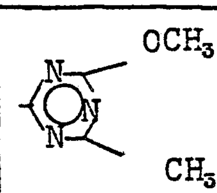 | |
| 157 | $CH_3(CH_2)_5CH{=}CH-$ | 0 | H | H | 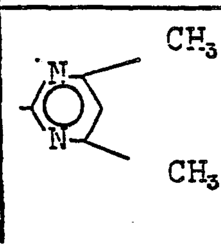 | Harz |

Tabelle 1 (Fortsetzung)

| Beispiel Nr. | $R_1$ | m | $R_2$ | $R_3$ | $R_4$ | Schmp. [°C] |
|---|---|---|---|---|---|---|
| 158 | $CH_3(CH_2)_5CH=CH-$ | 0 | H | H | 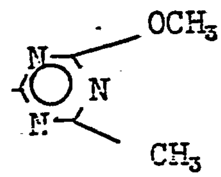 | Harz |
| 159 | $CH_3(CH_2)_6CHCH_2-$<br>Cl | 0 | H | H | 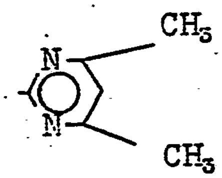 | |
| 160 | $CH_3(CH_2)_6CH=CH-$ | 0 | H | H | 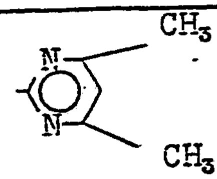 | |
| 161 | $CH_3(CH_2)_6CH=CH-$ | 0 | H | H | 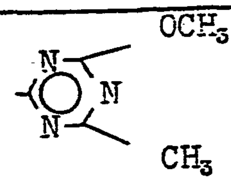 | |
| 162 | $CHF_2CF_2OCHCH_2-$<br>Cl | 0 | H | H | 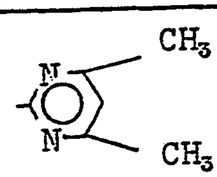 | 48-51 |
| 163 | $CHF_2CF_2OCHCH_2-$<br>Cl | 0 | H | H | 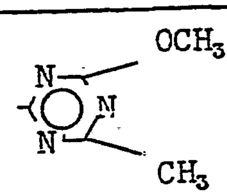 | 95-8 |
| 164 | $CH_3-CH-CH-$<br>Cl Cl | 0 | H | $CH_3$ | 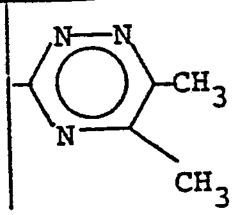 | 121-3 |

BAD ORIGINAL

0061661

## Formulierungsbeispiele

### Beispiel A

Ein emulgierbares Konzentrat wird erhalten aus:

15 Gew.-Teilen Wirkstoff
75 Gew.-Teilen Cyclohexan als Lösungsmittel
und 10 Gew.-Teilen oxethyliertes Nonylphenol (10 AeO) als Emulgator.

### Beispiel B

Ein in Wasser leicht dispergierbares benetzbares Pulver wird erhalten, indem man

25 Gew.-Teile Wirkstoff
64 Gew.-Teile kaolinhaltiges Quarz als Inertstoff
10 Gew.-Teile ligninsulfonsaures Kalium
und 1 Gew.-Teil oleylmethyltaurinsaures Natrium als Netz- und Dispergiermittel

mischt und in einer Stiftmühle mahlt.

### Beispiel C

Ein Stäubemittel wird erhalten, indem man

10 Gew.-Teile Wirkstoff
und 90 Gew.-Teile Talkum als Inertstoff

mischt und in einer Schlagmühle zerkleinert.

### Beispiel D

Ein Granulat besteht z. B. aus etwa

2 - 15 Gew.-Teilen Wirkstoff
98 - 85 Gew.-Teilen inerten Granulatmaterialien, wie z. B. Attapulgit, Bimsstein und Quarzsand.

BAD ORIGINAL

## Biologische Beispiele

### a) Herbizide Wirkung

### 1. Vorauflaufverfahren

Samen bzw. Rhizomstücke mono- und dikotyler Unkräuter wurden in Lehmerde in Plastiktöpfen (Ø 8 cm) ausgelegt und mit Erde abgedeckt. Die als benetzbare Pulver formulierten erfindungsgemäßen Verbindungen wurden in Form wäßriger Suspensionen oder Emulsionen auf die Erdoberfläche appliziert. Die Wasseraufwandmenge pro Topf entsprach dabei umgerechnet 600 - 800 l/ha. Nach der Behandlung wurden die Versuchstöpfe im Gewächshaus aufgestellt und die Versuchspflanzen unter guten Wachstumsbedingungen (Temperatur: 23 ± 1°C; rel. Luftfeuchte 60 - 80 %) kultiviert. Nach ca. 3 Wochen wurde die Pflanzenschädigung optisch bonitiert. Als Vergleich dienten dabei unbehandelte Kontrollen.

Die Schädigung der Unkrautpflanzen bzw. die Kulturpflanzenverträglichkeit wurde in einem Schlüssel von 0 - 5 ausgedrückt.

Dabei bedeutet
0 = ohne Wirkung (Schaden)
1 = 0 - 20 % Wirkung
2 = 20 - 40 % Wirkung
3 = 40 - 60 % Wirkung
4 = 60 - 80 % Wirkung
5 = 80 - 100 % Wirkung

Die Abkürzungen bedeuten:

LOM = Raygras
ECG = Hühnerhirse
STM = Sternmiere
AMR = Amaranth
SIA = Ackersenf
CYE = Sauergras (perennierend)
a.i.= Aktivsubstanz

In Tabelle 2 sind die Vorauflaufergebnisse zusammen gefaßt. Es wird deutlich, daß die erfindungsgemäßen Verbindungen eine gute herbizide Wirkung sowohl gegen monokotyle als auch gegen dikotyle Schadpflanzen aufweisen, wenn die Wirkstoffe im Vorauflaufverfahren appliziert werden.

Tabelle 2:

Vorauflaufwirkung gegen monokotyle und dikotyle Schadpflanzen. Wirkung in % im Vergleich zur unbehandelten Kontrolle.

| Beispiel Nr. | Dosis (kg a.i./ ha) | SIA | ECG | STM | LOM | AMR | CYE |
|---|---|---|---|---|---|---|---|
| 1 | 2,5 | 4 | 1 | 4 | 0 | 5 | 4 |
| | 10,0 | - | - | 5 | 1 | - | - |
| 5 | 2,5 | 4 | 1 | 5 | 1 | 3 | 5 |
| | 10,0 | - | - | 5 | 3 | - | - |
| 6 | 2,5 | 2 | 2 | 4 | 0 | 2 | 4 |
| 7 | 2,5 | 5 | 2 | 5 | 0 | 2 | 5 |
| 19 | 2,5 | 2 | 4 | 4 | 3 | 5 | - |
| 20 | 2,5 | 4 | 3 | 4 | 4 | 2 | 5 |
| | 10,0 | - | - | 4 | 5 | - | - |

Tabelle 2 (Fortsetzung)

| Beispiel Nr. | Dosis (kg a.i./ha) | SIA | ECG | STM | LOM | AMP. | CYE |
|---|---|---|---|---|---|---|---|
| 23 | 2,5 | 4 | 0 | 5 | 4 | - | - |
| 24 | 2,5 | 4 | 4 | 5 | 5 | - | - |
| 31 | 2,5 | 5 | 5 | 5 | 5 | 5 | 5 |
| 44 | 2,5 | 5 | 5 | 5 | 5 | - | - |
| 45 | 2,5 | 5 | 5 | 5 | 5 | - | - |
| 46 | 2,5 | 4 | 5 | 5 | 5 | - | - |
| 47 | 2,5 | 4 | 3 | 5 | 5 | - | - |
| 48 | 2,5 | 5 | 2 | 4 | 4 | - | - |
| 50 | 2,5 | 5 | 3 | 5 | 5 | - | - |
| 52 | 2,5 | 5 | 4 | 5 | 5 | - | - |
| 53 | 2,5 | 5 | 5 | 5 | 5 | - | - |
| 54 | 2,5 | 3 | 2 | 4 | 3 | - | - |
| 55 | 2,5 | 5 | 5 | 5 | 5 | - | - |
| 56 | 2,5 | 5 | 5 | 5 | 5 | 5 | 5 |
| 57 | 2,5 | 5 | 5 | 5 | 5 | 5 | 5 |
| 64 | 2,5 | 2 | 2 | 5 | 2 | - | - |
| 66 | 2,5 | 5 | 5 | 5 | 5 | - | - |
| 67 | 2,5 | 5 | 3 | 5 | 2 | - | - |
| 69 | 2,5 | 4 | 2 | 5 | 3 | - | - |
| 70 | 2,5 | 5 | 5 | 5 | 5 | - | - |
| 71 | 2,5 | 5 | 5 | 4 | 0 | - | - |
| 74 | 2,5 | 5 | 5 | 5 | 5 | - | - |
| 75 | 2,5 | 5 | 5 | 5 | 5 | - | - |
| 76 | 2,5 | 5 | 5 | 5 | 5 | - | - |
| 78 | 2,5 | 5 | 5 | 5 | 5 | 5 | 5 |
| 79 | 2,5 | 5 | 5 | 5 | 4 | 4 | 5 |
| 83 | 2,5 | 5 | 3 | 5 | 5 | - | - |
| 84 | 2,5 | 5 | 1 | 5 | 3 | - | - |
| 85 | 2,5 | 5 | 4 | 5 | 4 | - | - |
| 98 | 2,5 | 5 | 5 | 5 | 5 | - | - |
| 99 | 2,5 | 5 | 5 | 5 | 5 | - | - |
| 104 | 2,5 | 2 | 0 | 2 | 0 | - | - |

Tabelle 2 (Fortsetzung)

| Bei- spiel Nr. | Dosis (kg a.i./ ha) | SIA | ECG | STM | LOM | AMR | CYE |
|---|---|---|---|---|---|---|---|
| 106 | 2,5 | 5 | 5 | 5 | 5 | - | - |
| 107 | 2,5 | 5 | 5 | 5 | 5 | - | - |
| 115 | 2,5 | 5 | 5 | 5 | 5 | - | - |
| 116 | 2,5 | 5 | 5 | 5 | 5 | - | - |
| 118 | 2,5 | 5 | 5 | 5 | 5 | - | - |
| 120 | 2,5 | 5 | 5 | 5 | 5 | - | - |
| 123 | 2,5 | 4 | 2 | 3 | 2 | - | - |
| 136 | 2,5 | 5 | 5 | 5 | 5 | - | - |
| 138 | 2,5 | 5 | 5 | 5 | 5 | - | - |
| 139 | 2,5 | 2 | 3 | 4 | 4 | - | - |
| 141 | 2,5 | 5 | 0 | 4 | 3 | - | - |
| 143 | 2,5 | 5 | 5 | 5 | 5 | - | - |
| 148 | 2,5 | 5 | 3 | 5 | 5 | - | - |
| 149 | 2,5 | 5 | 3 | 5 | 4 | - | - |
| 150 | 2,5 | 5 | 4 | 5 | 5 | - | - |

2. Nachauflaufverfahren

Samen bzw. Rhizomstücke von mono- und dikotylen Unkräutern wurden in Töpfen ausgesät und im Gewächshaus unter guten Wachstumsbedingungen angezogen. 3 Wochen nach der Aussaat wurden die Versuchspflanzen im Dreiblattstadium behandelt.

Die als Spritzpulver bzw. als Emulsionskonzentrate formulierten erfindungsgemäßen Präparate wurden in verschiedenen Dosierungen auf die grünen Pflanzenteile gesprüht und nach ca. 3 Wochen Standzeit im Gewächshaus die Wirkung der Präparate optisch im Vergleich zu unbehandelten Kontrollen bonitiert.

Die erfindungsgemäßen Mittel weisen eine gute herbizide Wirksamkeit gegen ein breites wirtschaftlich wichtiger annueller und perennierender Unkräuter und Ungräser auf (Tabelle 3).

Tabelle 3:

Nachauflaufwirkung gegen monokotyle und dikotyle Schadpflanzen Wirkung in % im Vergleich zur unbehandelten Kontrolle.

| Beispiel Nr. | Dosis (kg a.i./ha) | SIA | ECG | STM | LOM | AMR | CYE |
|---|---|---|---|---|---|---|---|
| 1 | 2,5 | 5 | 1 | 4 | 1 | 0 | - |
| 5 | 2,5 | 5 | 1 | 4 | 1 | 0 | - |
| 6 | 2,5 | 4 | 1 | 3 | 1 | 2 | - |
| 7 | 2,5 | 1 | 1 | 3 | 1 | 2 | - |
| 19 | 2,5 | 2 | 1 | 3 | 1 | 3 | - |
| 20 | 2,5 | 5 | 1 | 4 | 2 | 1 | - |
| 23 | 2,5 | 3 | 0 | 3 | 0 | - | - |

 0061661

Tabelle 3 (Fortsetzung)

| Beispiel Nr. | Dosis (kg a.i./ha) | SIA | ECG | STM | LOM | AMR | CYE |
|---|---|---|---|---|---|---|---|
| 24 | 2,5 | 3 | 2 | 4 | 3 | - | - |
| 31 | 2,5 | 5 | 4 | 5 | 5 | 5 | - |
| 44 | 2,5 | 5 | 5 | 5 | 5 | - | - |
| 45 | 2,5 | 5 | 5 | 5 | 5 | - | - |
| 46 | 2,5 | 4 | 0 | 4 | 3 | - | - |
| 47 | 2,5 | 5 | 4 | 4 | 3 | - | - |
| 48 | 2,5 | 5 | 0 | 4 | 3 | - | - |
| 50 | 2,5 | 3 | 0 | 3 | 2 | - | - |
| 52 | 2,5 | 5 | 0 | 5 | 5 | - | - |
| 53 | 2,5 | 5 | 3 | 5 | 4 | - | - |
| 54 | 2,5 | 3 | 2 | 2 | 3 | - | - |
| 55 | 2,5 | 4 | 5 | 4 | 4 | - | - |
| 56 | 2,5 | 5 | 4 | 5 | 4 | 5 | 0 |
| 57 | 2,5 | 5 | 3 | 5 | 4 | 5 | 0 |
| 64 | 2,5 | 4 | 2 | 4 | 0 | - | - |
| 66 | 2,5 | 5 | 3 | 5 | 4 | - | - |
| 67 | 2,5 | 3 | - | 2 | 2 | - | - |
| 69 | 2,5 | 5 | - | 4 | 2 | - | - |
| 70 | 2,5 | 4 | 2 | 4 | 2 | - | - |
| 71 | 2,5 | 4 | 2 | 4 | 2 | - | - |
| 74 | 2,5 | 5 | 5 | 5 | 5 | - | - |
| 75 | 2,5 | 5 | 5 | 5 | 5 | - | - |
| 76 | 2,5 | 5 | 5 | 5 | 5 | - | - |
| 78 | 2,5 | 5 | 4 | 5 | 4 | 5 | - |
| 79 | 2,5 | 5 | 5 | 5 | 4 | 5 | - |

Tabelle 3 (Fortsetzung)

| Bei-spiel Nr. | Dosis (kg a.i./ ha) | SIA | ECG | STM | LOM | AMR | CYE |
|---|---|---|---|---|---|---|---|
| 83 | 2,5 | 4 | 2 | 3 | 2 | - | - |
| 84 | 2,5 | 5 | 1 | 5 | 0 | - | - |
| 85 | 2,5 | 5 | 2 | 4 | 3 | - | - |
| 98 | 2,5 | 4 | 4 | 3 | 3 | - | - |
| 99 | 2,5 | 4 | 5 | 5 | 4 | - | - |
| 104 | 2,5 | 4 | 2 | 2 | 1 | - | - |
| 105 | 2,5 | 5 | 3 | 4 | 3 | - | - |
| 106 | 2,5 | 5 | 5 | 5 | 5 | - | - |
| 107 | 2,5 | 5 | 5 | 5 | 5 | - | - |
| 115 | 2,5 | 5 | 5 | 5 | 5 | - | - |
| 116 | 2,5 | 5 | 4 | 5 | 5 | - | - |
| 118 | 2,5 | 5 | 5 | 5 | 5 | - | - |
| 120 | 2,5 | 5 | 5 | 5 | 5 | - | - |
| 123 | 2,5 | 2 | 2 | 4 | 3 | - | - |
| 136 | 2,5 | 5 | 3 | 4 | 3 | - | - |
| 138 | 2,5 | 5 | 5 | 5 | 5 | - | - |
| 139 | 2,5 | 5 | 5 | 4 | 3 | - | - |
| 141 | 2,5 | 5 | 3 | 3 | 2 | - | - |
| 143 | 2,5 | 5 | 4 | 4 | 5 | - | - |
| 148 | 2,5 | 5 | 1 | 2 | 3 | - | - |
| 149 | 2,5 | 5 | 3 | 3 | 3 | - | - |
| 150 | 2,5 | 5 | 2 | 4 | 3 | - | - |

 

b) Pflanzenwuchsregulierende Wirkung

Beispiel I (Wuchshemmung an Getreide)

In Schalenversuchen im Gewächshaus wurden junge Getreidepflanzen (Weizen, Gerste und Roggen) im 3-Blatt-Stadium mit der in Tabelle 1 genannten Verbindung in den angegebenen Wirkstoffkonzentrationen (kg/ha) tropfnaß gespritzt. Als Vergleichsverbindungen wurde 2-Chloräthyltrimethylammoniumchlorid eingesetzt. Nachdem die unbehandelten Kontrollpflanzen eine Wuchshöhe von etwa 55 cm erreicht hatten, wurde bei allen Pflanzen der Zuwachs gemessen und die Wuchshemmung in % des Zuwachses der Kontrollpflanzen berechnet. Es wurde außerdem die phytotoxische Wirkung der Verbindungen beobachtet. Die Ergebnisse sind in der Tabelle I zusammengefaßt. Bei der Angabe der Wuchshemmung bedeuten 100 % den Stillstand des Wachstums und 0 % ein Wachstum entsprechend dem der unbehandelten Kontrollpflanzen.

Tabelle 4

Wuchshemmung bei Getreide

| Verbindung gemäß Beispiel | Anwendungskonzentration (kg/ha) | Wuchshemmung in % Weizen | Gerste | Roggen | Phytotoxische Wirkung |
|---|---|---|---|---|---|
| 56 | 0,62<br>0,31 | 34<br>32 | 32<br>28 | 27<br>25 | keine Schäden |
| 31 | 0,62<br>0,31 | 34<br>23 | 27<br>24 | 24<br>19 | keine Schäden |
| Vergleich (2-Chloräthyl)-trimethylammoniumchlorid | 2,5<br>1,25 | 28<br>20 | 8<br>0 | 9<br>0 | keine Schäden |

## Beispiel II (Wuchshemmung an Soja- und Buschbohne)

10 - 15 cm hohe Soja- bzw. Buschbohnen wurden mit den Wirkstoffzubereitungen tropfnaß bespritzt. Nach 2 Wochen wird der Zuwachs gemessen und die Wuchshemmung in % des Zuwachses der Kontrollpflanzen berechnet. Die Ergebnisse sind in Tabelle 5 zusammengefaßt.

Tabelle 5

Wuchshemmung an Soja- und Buschbohne

| Verbindung gemäß Beispiel | Anwendungskonzentration (kg/ha) | Wuchshemmung in % Soja | Wuchshemmung in % Buschbohne | Phytotoxische Wirkung |
|---|---|---|---|---|
| 56 | 0,62 | 23 | 42 | keine Schäden |
| | 0,31 | 21 | 39 | |
| 31 | 0,62 | 24 | 37 | keine Schäden |
| 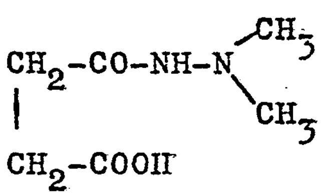 (Aminozide) | 2,5 | 12 | 34 | keine Schäden |

0061661

## Beispiel III (Wuchshemmung an Rasen)

Eine Rasenmischung, bestehend aus 5 repräsentativen Arten, wird nach 3maligem Rückschnitt mit einer Wirkstoffzubereitung tropfnaß gespritzt. Nach 3 - 4 Wochen wird der Zuwachs gemessen und die Wuchshemmung in % des Zuwachses der Kontrollpflanzen berechnet. Es bedeuten 100 % den Stillstand des Wachstums und 0 % ein Wachstum entsprechend dem der unbehandelten Kontrollpflanzen.

Tabelle 6

Wuchshemmung an Rasen

| Verbindung gemäß Beispiel | Anwendungskonzentration (kg/ha) | Wuchshemmung in % | Phytotoxische Wirkung |
|---|---|---|---|
| 56 | 0,62 | 54 | keine |
| | 0.31 | 37 | Schäden |
| 31 | 0.62 | 45 | keine |
| | 0.31 | 42 | Schäden |
| Maleinsäurehydrazid | 2.5 | 55 | starke Schäden |

Beispiel IV (Erhöhung des Zuckergehaltes bei Zuckerrohr)

Untersuchungsverfahren

Zuckerrohrpflanzen werden unter Gewächshausbedingungen bei 25°C - 35°C und ca. 65 % Luftfeuchtigkeit angezogen. Unterschiedliche Mengen der formulierten Mittel wurden in Wasser suspendiert, daß zusätzlich etwa 0,25 Gew.-% eines oberflächenaktiver Mittels (Nonylphenol) enthielt.
Jeweils 0,3 ml der Suspensionen wurden mit Hilfe einer Spritze in den Spindelbereich in Höhe der letzten sichtbaren Blattspreite ("dewlap") appliziert (10 Pflanzen pro Konzentration). Von den behandelten Pflanzen sowie von den nicht behandelten Kontrollen wurden nach 3 Wochen bei der Ernte die Blätter entfernt und die Internodien gruppenweise auf ihren Saccharosegehalt analysiert. Die Ergebnisse sind in Tabelle 7 dargestellt.

Tabelle 7

| Verbindung gemäß Beispiel | Anwendungskonzentration (kg/ha) | Zuckergehalt (%) bei der Ernte |
|---|---|---|
| 56 | 0,62 | 164 |
| 31 | 0,62 | 181 |
| Kontrolle | | 100 % |

0061661

Beispiel V

Abszissionswirkung bei Citruspflanzen

Astpartien von Orangenbäumen (Sorten Hamlin, Pineapple und Valencia) mit mindestens 20 Früchten wurden kurz vor der Ernte mit Wirkstofflösungen bespritzt. Nach 7 Tagen erfolgte die Auswertung durch Messung der Haltekraft.

Tabelle 8

| Bsp.-Nr. | Dosierung | Haltekraft (kg) |
|---|---|---|
| Kontrolle | | 6,5 |
| 5-Chlro-3-methyl-4-nitro-pyrazol (Vergleich) | | 1,6 |
| 57 | 1000 | 1,4 |
| 79 | 1000 | 0 |
| 106 | 1000 | 1,9 |

Die vorgenannten Verbindungen zeigten eine hervorragende Pflanzenverträglichkeit an Citrusbäumen.

Sie führten zu keinerlei Schäden an den Blättern und den unreifen Früchten. Im Vergleich zum technischen Standard führten die Substanzen zu weitaus geringeren Schalenverletzungen der reifen Früchte (zumeist überhaupt nicht sichtbar). Über die Schalenverletzung wird bekannterweise von Citrusabscissionsmitteln eine Wundäthylenfreisetzung induziert, die eine Ausbildung des Trenngewebes fördert und so zum Fruchtfall führt.

Patentansprüche

1. Verbindungen der Formel I

$$R_1-(O)_m-SO_2-\underset{R_2}{N}-\overset{X}{\overset{\|}{C}}-\underset{R_3}{N}-R_4 \qquad (I)$$

worin

$R_1$ einen gegebenenfalls durch bis zu 6 Halogematome substituierten gesättigten oder ungesättigten, verzweigten oder unverzweigten aliphatischen Rest mit bis zu 10 C-Atomen, der ggf. durch Sauerstoff unterbrochen sein kann,

$R_2$, $R_3$ H oder $(C_1-C_4)$-Alkyl

X O oder S;

m 0 oder 1;

$R_4$ einen 2-3 Stickstoffatome enthaltenden heterocyclischen Sechsring, der ggf. 1-3 fach durch Halogen, $NO_2$, CN, CHO, $(C_1-C_4)$-Alkylamino, $(C_1-C_4)$Dialkylamino, einen (gegebenenfalls durch Halogen, $(C_1-C_3)$-Alkoxy, $(C_1-C_3)$-Alkylthio, $(C_1-C_3)$-Alkylamino, $(C_1-C_3)$-Dialkylamino oder $(C_1-C_4)$Alkoxycarbonyl substituierten) $(C_1-C_4)$-Alkylrest, einen (gegebenenfalls durch Halogen oder $(C_1-C_4)$Alkoxycarbonyl substituierten) $(C_1-C_4)$-Alkoxy- oder $(C_1-C_4)$-Alkylthiorest oder $(C_1-C_4)$-Alkoxycarbonyl substituiert ist,

bedeuten, sowie, falls $R_2$ Wasserstoff bedeutet, deren physiologisch verträgliche Salze mit Basen.

2. Verfahren zur Herstellung von Verbindungen der Formel I, dadurch gekennzeichnet, daß man

a) Verbindungen der Formel

$$R_1 - (O)_m - SO_2 - N = C = O \qquad (II),$$

oder

$$R_1-(O)_m-SO_2-\underset{R_2}{\underset{|}{N}}-\overset{X}{\overset{\|}{C}}-Cl \qquad (III)$$

mit Verbindungen der Formel

$$\underset{R_3}{\underset{|}{HN}}-R_4 \qquad (IV)$$

oder

b) Verbindungen der Formel

$$R_1-(O)_m-SO_2-\underset{R_2}{\underset{|}{NH}} \qquad (V)$$

mit Verbindungen der Formel

$$S=C=N-R_4 \qquad (VI)$$

oder

$$Cl-\overset{X}{\overset{\|}{C}}-\underset{R_2}{\underset{|}{N}}-R_4 \qquad (VII)$$

umsetzt, wobei in Formel VII $R_3$ für $(C_1-C_4)$-Alkyl steht, und gewünschtenfalls die erhaltenen Verbindungen der Formel I durch Abspaltung von Halogenwasserstoff, Anlagerung von Halogen an vorhandene Mehrfachbindungen, Alkylierung in $R_2$-Position oder Salzbildung in andere Verbindungen der Formel I überführt.

3. Herbizide und wachstumsregulierende Mittel, gekennzeichnet durch einen Gehalt an Verbindungen der Formel I.

4. Verwendung von Verbindungen der Formel I zur Bekämpfung von unerwünschtem Pflanzenwuchs und zur Wachstumsregulierung.

5. Verfahren zur Bekämpfung von unerwünschtem Pflanzenwuchs

bzw. zur Wachstumsregulierung, dadurch gekennzeichnet, daß man auf die zu behandelnden Pflanzen oder die Anbaufläche eine wirksame Menge einer Verbindung der Formel I aufbringt.

Patentansprüche für Österreich

1. Verfahren zur Herstellung von Verbindungen der Formel I,

$$R_1-(O)_m-SO_2-\underset{R_2}{N}-\overset{X}{\overset{\|}{C}}-\underset{R_3}{N}-R_4 \qquad (I)$$

worin

$R_1$ einen gegebenenfalls durch bis zu 6 Halogenatome substituierten gesättigten oder ungesättigten, verzweigten oder unverzweigten aliphatischen Rest mit bis zu 10 C-Atomen, der ggf. durch Sauerstoff unterbrochen sein kann,

$R_2$, $R_3$ H oder $(C_1-C_4)$-Alkyl

X O oder S,

m 0 oder 1;

$R_4$ einen 2-3 Stickstoffatome enthaltenden heterocyclischen Sechsring, der ggf. 1-3 fach durch Halogen, $NO_2$, CN, CHO, $(C_1-C_4)$-Alkylamino, $(C_1-C_4)$-Dialkylamino, einen (gegebenenfalls durch Halogen, $(C_1-C_3)$-Alkoxy, $(C_1-C_3)$-Alkylthio, $(C_1-C_3)$-Alkylamino, $(C_1-C_3)$-Dialkylamino oder $(C_1-C_4)$Alkoxycarbonyl substituierten) $(C_1-C_4)$-Alkylrest, einen (gegebenenfalls durch Halogen oder $(C_1-C_4)$Alkoxycarbonyl substituierten) $(C_1-C_4)$-Alkoxy- oder $(C_1-C_4)$-Alkylthiorest oder $(C_1-C_4)$-Alkoxycarbonyl substituiert ist,

bedeuten, sowie, falls $R_2$ Wasserstoff bedeutet, deren physiologisch verträgliche Salze mit Basen,

dadurch gekennzeichnet, daß man

a) Verbindungen der Formel

$$R_1 - (O)_m - SO_2 - N = C = O \qquad (II),$$

oder

$$R_1 - (O)_m - SO_2 - \underset{R_2}{N} - \overset{X}{\overset{\|}{C}} - Cl \qquad (III)$$

mit Verbindungen der Formel

$$\underset{\displaystyle R_3}{HN} - R_4 \qquad (IV)$$

oder

b) Verbindungen der Formel

$$R_1 - (O)_m - SO_2 - \underset{\displaystyle R_2}{NH} \qquad (V)$$

mit Verbindungen der Formel

$$S = C = N - R_4 \qquad (VI)$$

oder

$$Cl - \overset{\displaystyle X}{\overset{\|}{C}} - \underset{\displaystyle R_2}{N} - R_4 \qquad (VII)$$

umsetzt, wobei in Formel VII $R_3$ für $(C_1-C_4)$-Alkyl steht, und gewünschtenfalls die erhaltenen Verbindungen der Formel I durch Abspaltung von Halogenwasserstoff, Anlagerung von Halogen an vorhandene Mehrfachbindungen, Alkylierung in $R_2$-Position oder Salzbildung in andere Verbindungen der Formel I überführt.

2. Herbizide und wachstumsregulierende Mittel, gekennzeichnet durch einen Gehalt an Verbindungen der Formel I.

3. Verwendung von Verbindungen der Formel I zur Bekämpfung von unerwünschtem Pflanzenwuchs und zur Wachstumsregulierung.

4. Verfahren zur Bekämpfung von unerwünschtem Pflanzenwuchs bzw. zur Wachstumsregulierung, dadurch gekennzeichnet, daß man auf die zu behandelnden Pflanzen oder die Anbaufläche eine wirksame Menge einer Verbindung der Formel I aufbringt.

0061661

Europäisches Patentamt

# EUROPÄISCHER RECHERCHENBERICHT

Nummer der Anmeldung

EP 82 10 2200

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| D,A | DE-A-2 715 786 (E.I. DU PONT DE NEMOURS AND COMPANY)<br>* Patentansprüche *<br>----- | | C 07 D 251/16<br>C 07 D 251/22<br>C 07 D 251/46<br>C 07 D 239/42<br>C 07 D 239/46<br>C 07 D 239/52<br>C 07 D 253/06<br>A 01 N 47/36 |
| | | | **RECHERCHIERTE SACHGEBIETE (Int. Cl. 3)** |
| | | | C 07 D 251/00<br>C 07 D 239/00<br>C 07 D 253/00 |

Der vorliegende Recherchenbericht wurde für alle Patentansprüche erstellt.

| Recherchenort | Abschlußdatum der Recherche | Prüfer |
|---|---|---|
| DEN HAAG | 29-06-1982 | VAN BIJLEN H. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503. 03.82